# EUROPÄISCHE PATENTSCHRIFT

(11) **EP 0 932 386 B1**
(45) Veröffentlichungstag und Bekanntmachung des Hinweises auf die Patenterteilung: **08.09.2004**
(21) Anmeldenummer: 97947685.0
(22) Anmeldetag: 17.10.1997
(51) Int. Cl.: A61K 7/027, A61K 7/48

(54) **KOSMETISCHE UND DERMATOLOGISCHE STIFTE MIT HOHEM WASSERGEHALT**
COSMETIC AND SKIN CARE STICKS WITH HIGH WATER CONTENTS
BATONS DE COSMETIQUE ET DE SOINS DERMATOLOGIQUES A FORTE TENEUR EN EAU

(30) Priorität: 19.10.1996 DE 19643237
(43) Veröffentlichungstag der Anmeldung: 04.08.1999
(73) Patentinhaber: Beiersdorf Aktiengesellschaft, 20245 Hamburg (DE)
(72) Erfinder: DIEC, Khiet, Hien, D-22523 Hamburg (DE); KLIER, Manfred, D-21521 Aumühle (DE); SCHREIBER, Jörg, D-22087 Hamburg (DE); WOLF, Florian, D-20251 Hamburg (DE)
(86) Internationale Anmeldenummer: PCT/DE1997/002401
(87) Internationale Veröffentlichungsnummer: WO 1998/017232

(56) Entgegenhaltungen:
- EP-A- 0 522 624
- WO-A-93/04658
- DE-A- 2 335 549
- US-A- 4 725 431
- PATENT ABSTRACTS OF JAPAN vol. 003, no. 075 (C-050), 27.Juni 1979 & JP 54 049337 A (KANEBO LTD), 18.April 1979,
- G. PROSERPIO: "Stabilisation des émulsions" PARFUMS COSMET. AROMES, Nr. 39, 1981, Seiten 71-75, XP002057440
- W.E. ADAM: "Neue Polyalkylenglykol-Copolymere für die Kosmetik" SEIFEN ÖLE FETTE WACHSE, Bd. 110, Nr. 15, 1984, Seiten 427-431, XP002057441
- D. BOUTET: "Nouveaux glycols copolymères utilisés en cosmétique" PARFUMS COSMET. AROMES, Nr. 54, 1983, Seiten 49-53, XP002057442

## Beschreibung

Die vorliegende Erfindung betrifft kosmetische Stifte, welche sich durch einen hohen Wassergehalt auszeichnen, und welche als vorteilhafte Ausführungsformen W/O-Emulsionen darstellen können. Insbesondere betrifft die vorliegende Erfindung Lippenstifte, bevorzugt Lippenpflegestifte, aber auch dekorative Lippenstifte, ferner Stiftformulierungen welche beispielsweise zur Verwendung gegen Akne geeignet sind. Als weitere vorteilhafte Ausführungsformen betrifft die vorliegende Erfindung Sonnenschutzstifte, Lidschattenstifte und vergleichbare Produkte.

Technisch betrachtet, sind die meisten Stiftformulierungen wasserfreie Fettmischungen aus festen oder halbfesten Wachsen und flüssigen Ölen, wobei die hochgereinigten Paraffinöle und -wachse die Lippenstiftgrundmasse darstellen. Auch wasserhaltige Zubereitungen sind bekannt, welche gelegentlich auch in Form von W/O-Emulsionen vorliegen.

Nach dem idealen Anforderungsprofil sollen sich kosmetische oder pharmazeutische Stifte glatt und ohne großen Reibungswiderstand auftragen lassen. Darüber hinaus muß eine solche Formulierung auch noch die Anforderungen erfüllen, daß der betreffende Stift bruchfest und temperaturbeständig sein muß und die Formulierung nicht ausölen darf.

Ein Lippenstift im besonderen soll schon bei leichtem Andruck einen nicht schmierigen, stumpfen oder klebrigen, aber dennoch gut haftenden Fettfilm an die Lippen abgeben. Durch diesen Fettfilm sollen die Lippen dann glatt und geschmeidig gemacht werden.

Sollen kosmetische oder pharmazeutische Stifte bestimmte Wirkstoffe enthalten, ist denkbar, daß die übrigen Bestandteile mit den Wirkstoffen nicht kompatibel sind. Dies ist besonders häufig der Fall, wenn die Verwendung der kosmetischen Stifte als Sonnenschutzstifte vorgesehen ist, und insbesondere wasserlösliche Lichtschutzfilter in größeren Mengen im Stift enthalten sein sollen oder wenn zur Herstellung eines Antiakne-Stifts wasserlöslichen Antiakne-Wirkstoffe in dem Fachmann bekannten Mengen eingearbeitet werden sollen.

Aus Gründen der Verträglichkeit ist es stets zu bevorzugen, selbst bei Verwendung an sich unbedenklicher Substanzen, entsprechende Einsatzkonzentrationen solcher Wirkstoffe möglichst niedrig zu halten.

Eine Aufgabe der vorliegenden Erfindung war es also, Zubereitungen zu entwickeln, welche als Grundlage für chemische Lichtschutzfilter, physikalische Lichtschutzfilter, Antiakne-Wirkstoffe, Vitamine geeignet sind, und die Nachteile des Standes der Technik nicht aufweisen. Weiterhin war es also eine Aufgabe der Erfindung, kosmetische Grundlagen zu entwickeln, die sich durch gute Hautverträglichkeit auszeichnen.

Übliche Grundstoffe des Standes der Technik für stiftförmige Zubereitungen sind beispielsweise flüssige Öle (z.B. Paraffinöle, Ricinusöl, Isopropylmyristat), halbfeste Bestandteile (z.B. Vaseline, Lanolin), feste Bestandteile (z.B. Bienenwachs,, Ceresin und Mikrokristalline Wachse bzw. Ozokerit) hochschmelzende Wachse (z.B. Carnaubawachs, Candelillawachs)

Lippenstifte des Standes der Technik mit einem Gehalt an Paraffinen und Bienenwachs sind in "Kosmetik, Entwicklung Herstellung und Anwendung kosmetischer Mittel", S. 105 , Herausgeber: W.Umbach, Georg Thieme Verlag, Stuttgart - New York, 1988, beschrieben.

Der Stand der Technik hat aber eine Reihe von Nachteilen. Dazu zählt die Tatsache, daß wasserlösliche Wirkstoffe häufig nicht gut genug fettlöslich sind, als daß sie in nennenswertem Maße in die kosmetischen Grundlagen einzubauen wären. Andererseits wäre ein gewisser Wassergehalt durchaus erwünscht, um die Kompatibilität des kosmetischen Stiftes mit der menschlichen Haut zu erhöhen. Ferner sind Stifte mit sehr hohen Wasseranteilen nach dem Stand der Technik deshalb nicht machbar, weil das Wasser mit der hydrophoben Öl/Wachs/Emulgator-Matrix nicht kompatibel ist.

Für einen Antiakne-Stift beispielsweise wäre es aber gerade besonders vorteilhaft, wenn der Anteil an fettlöslichen Bestandteilen möglichst niedrig läge.

Die Haut der Lippen besitzt nur eine äußerst dünne Homschicht. Schweißdrüsen sind auf den Lippen gar nicht, Talgdrüsen nur vereinzelt zu finden. Daher ist die Lippenhaut praktisch frei von Fett und neigt, besonders bei kaltem und trockenem Wetter, zum Austrocknen. Dabei können sich kleine Risse in der Haut bilden, und die Empfindlichkeit der Lippen gegenüber chemischen, physikalischen und mikrobiellen Einwirkungen (z.B. Nahrungsmittel, Sonnenlicht, Herpes-Simplex-Viren) steigt.

Dies zu verhindern ist die Aufgabe von Lippenpflegestiften. Diese Produkte enthalten meist zu einem hohen Anteil Wachse und Fettkomponenten, die nach dem Auftragen eine abdeckende Schicht über den Lippen ausbilden.

In die Zubereitungen für Lippenpflegestifte können zusätzlich Wirkstoffe eingearbeitet werden, die der Lippenpflege oder dem Lippenschutz förderlich sind, z.B. Vitamine, Feuchtigkeit spendende Mittel, Lichtschutzmittel, abdeckende Pigmente usw.

Die Lederhaut der Lippen weist gut durchblutete Papillen auf, die bis dicht unter die Lippenoberfläche reichen. Daher sind die Lippen rötlich gefärbt und, je nach Teintfarbe der betreffenden Person, von der übrigen Gesichtshaut mehr oder weniger stark farblich abgesetzt. Ein Stilmittel der dekorativen Kosmetik ist dann auch, die Lippenfarbe durch entsprechende Kosmetika auf den Typ der Person abzustimmen.

Produkte dieser Art sind dekorative Lippenstifte, in welche verschiedenste Farbpigmente eingearbeitet werden können. Auch diese Stifte enthalten zu hohen Anteilen Wachse und Fettkomponenten, die nach dem Auftragen eine abdeckende Lipidschicht über den Lippen ausbilden.

Die Aufgabe dieser Schicht ist jedoch nicht vorderhand, die Lippenhaut vor dem Austrocknen zu schützen. Die Lipidschicht dient hier als auf den Lippen haftende Grundlage für die eingearbeiteten Pigmentstoffe; die Pigmente selbst können aus mancherlei Gründen nicht ohne eine solche Grundlage auf die Lippen aufgetragen werden.

Es ist auch möglich, die Eigenschaften der pflegenden und dekorativen Lippenstifte miteinander zu kombinieren, d.h., in dekorative Lippenstifte pflegende oder schützende Substanzen einzuarbeiten.

Da sowohl pflegende als auch vorwiegend dekorative Lippenstifte des Standes der Technik teilweise gravierende Mängel aufweisen, war eine weitere Aufgabe der vorliegenden Erfindung, diesen Mängeln Abhilfe zu schaffen.

Wegen der hohen Empfindlichkeit des Lippenbereiches, insbesondere gegenüber ultravioletter Strahlung infolge des praktisch völligen Mangels an Pigmenten, empfiehlt sich, zumal bei erhöhter UV-Exposition wie im Hochgebirge, dem Lippenbereich einen Schutz gegen UV-Strahlung in Form von entsprechenden stiftförmigen Lichtschutzzubereitungen zukommen zulassen. Gerade in stiftförmigen Zubereitungen des Standes der Technik werden oft anorganischen Pigmente als UV-Absorber bzw. UV-Reflektoren zum Schutze des Lippenbereiches vor UV-Strahlen verwendet. Dabei handelt es sich insbesondere um Oxide des Titans, aber auch gelegentlich des Zinks, Eisens, Zirkoniums, Siliciums, Mangans, Aluminiums, Cers und Mischungen davon, sowie Abwandlungen.

Ein erheblicher Mangel der Formulierungen des Standes der Technik besteht unter anderem darin, daß es wegen der niedrigen Wassergehalte an sich akzeptabler Emuisionsstifte praktisch unmöglich war, wasserlösliche UV-Filtersubstanzen in solche Formulierungen einzuarbeiten. Eine weitere Aufgabe der vorliegenden Erfindung war also, Stifte mit ausschließlich wasserlöslichen UV-Filtem oder wasserdispergier-baren Pigmenten (zum Beispiel Titandioxid) zugänglich zu machen beziehungsweise Kombinationen aus wasserlöslichen und fettlöslichen UV-Filtern.

Aus dem DBP 23 35 549 ist ein Verfahren zur Herstellung eines kosmetischen Stiftes auf der Basis einer W/O-Emulsion bekannt. Nach dieser Lehre wird aus einer Polyhydroxyverbindung und einer nichtionogenen, oberflächenaktiven Verbindung ein Gel hergestellt, dieses mit einer kosmetischen Grundlage vermischt und Wasser in die Mischung emulgiert.

Nach diesem Verfahren sind jedoch keine Stifte herzustellen, die über die gestellten universellen Anforderungen an einen kosmetischen Stift verfügen. Da dieses Verfahren darüberhinaus kein Ein-Schritt-Verfahren darstelt, zeichnet es sich durch weitere Nachteile aus.

Die DE-OS 41 28 748 beschreibt kosmetische Stifte, weiche dadurch gekennzeichnet sind, daß sie Emulsionen darstellen und als wesentliche Bestandteile Bienenwachs, einen oder mehrere Ester aus einer gesättigten Carbonsäure mit 20 - 40 Kohlenstoffatomen und einem gesättigten Alkohol mit 14 - 34 Kohlenstoffatomen, Wasser, sowie gegebenenfalls weitere Lipide und/oder übliche Hilfs- und Zusatzstoffe enthalten. Obwohl diese Zubereitungen zwar vorteilhafte Eigenschaften haben, sind doch noch gewisse Nachteile in Kauf zu nehmen.

Die US-PS 4,719,103 beschreibt einen Antitranspirantstift auf der Basis einer W/O-Emulsion, welcher einen hohen Wasseranteil enthalte, welcher sich auszeichnet durch einen Gehalt an flüchtigen Silikonkomponenten, ein festes Alkanol sowie Polyglycerinfettsäureester, beispielsweise Polyglycerylisostearat, als Emulgator. Die US-PS 4,704,271 und die US-PS 4,725,431 beschreiben ähnliche Zubereitungen.

Die GB-OS 2 162 439 beschreibt paraffinhaltige Stifte, welche einen hohen Wasseranteil enthalten sollen, wobei die Emulgatoren aus der Gruppe der Metallsalze gewählt werden.

Es war nach all diesem überraschend und nicht vorhersehbar, daß kosmetische Stifte, insbesondere solche, gewählt aus der Gruppe der Lippenstifte, Antiaknestifte, Sonnenschutzstifte und Lidschattenstifte, dadurch gekennzeichnet, daß sie
(a) eine Fettphase , welche
   (a1) mindestens eine Ölkomponente
   (a2) mindestens eine Wachskomponente
   (a3) gegebenenfalls weitere in der Fettphase lösliche oder dispergierbare Substanzen
   umfaßt:
(b) eine Wasserphase, welche
   (b1) 50 bis 85 Gew.% Wasser bezogen auf das Gesamtgewicht der Stiftfüllgutmasse sowie
   (b2) gewünschtenfalls in Wasser lösliche oder dispergierbare Substanzen umfaßt,
(c) gewünschtenfalls einen oder mehrere Wirkstoffe, gewählt aus der für Lippenstifte, Antiaknestifte, Sonnenschutzstifte und Lidschattenstifte bekannten Wirkstoffe,
d) mindestens einen W/O-Emulgator oder ein Gemisch aus mehreren W/o-Emulgatoren,
(e) eine oder mehrere Stabilisatoren, gewählt aus der Gruppe der Substanzen der allgemeinen Struktur A-B-A', wobei A und A' gleiche oder verschiedene hydrophobe organische Reste darstellen und B eine hydrophile Gruppe bedeutet,
(f) gewünschtenfalls weitere grenzflächenaktive Substanzen als Coemulgatoren enthalten, ferner gewünschtenfalls weitere Stabilisatoren und weitere übliche kosmetische und/oder pharmazeutische Hilfs-, Wirk- und/oder Zusatzstoffe,
   enthalten,
   die Nachteile des Standes der Technik beseitigen.

Es war erstaunlich, daß die erfindungsgemäßen Zubereitungen die Einarbeitung hoher Wassermengen, selbst bei Gegenwart nur geringer Mengen an erfindungsgemäß verwendeten Emulgatoren erlaubt. Die Freisetzung insbesondere wasserlöslicher Wirkstoffe ist gegenüber den herkömmlicher Zubereitungen deutlich erhöht. Ein Beispiel ist die Steigerung des Lichtschutzfaktors, welches erfindungsgemäß in geringerer Konzentration besser wirksam ist als die Zubereitungen des Standes der Technik also zum Beispiel im Vergleich zu W/O-Stiften mit niedrigem Wassergehalt oder im Vergleich zu wasserfreien Suspensionstiften.

Aber auch die kosmetischen Eigenschaften der erfindungsgemäßen wasserreichen Stifte erweisen sich gegenüber denen des Standes der Technik deutlich verbessert. Beispielsweise läßt sich selbst ohne weitere Zusätze eine angenehme Kühlwirkung auf der Haut durch bloßes Auftragen erzielen, was sich insbesondere bei der Verwendung als Sonnenschutzstift, Antiaknestift und Lippenstift angenehm bemerkbar macht.

Auch bei der Verwendung als Lippenstift, Befeuchtung der Haut oder Antiaknestift machen sich deutliche Verbesserungen gegenüber dem Stande der Technik bemerkbar. Es ist beispielsweise zur Herstellung dieser Stifte möglich, wasserdispergierbares Titandioxid einzusetzen.

Die Herstellung erfindungsgemäßer Stifte gemäß Anspruch 2 ist dabei sehr einfach, da es sich um ein Ein-Schritt-Verfahren handelt, bei der beispielsweise die Wasserphase zur heißen Fettphase gegeben und anschließend auf Raumtemperatur abgekühlt wird.

Ferner zeichnet sich das erfindungsgemäße Verfahren dadurch aus, daß zur Herstellung der erfindungsgemäßen Stifte eine Vielzahl von Emulgatoren beziehungsweise Ölkomponenten eingesetzt werden können.

Der erfindungsgemäß verwendete W/O-Emulgator bzw. die W/O-Emulgatoren aus der Substanzgruppe A-B-A' wird oder werden erfindungsgemäß vorteilhaft gewählt aus der Gruppe der Substanzen der allgemeinen Formel 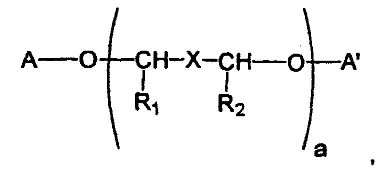 wobei
- A und A' gleiche oder verschiedene hydrophobe organische Reste darstellen,
- a eine Zahl von 1 bis 100, vorzugsweise 2 bis 60, insbesondere 5 bis 40 darstellt,
- X eine Einfachbindung oder die Gruppe 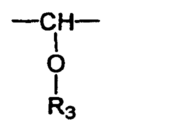 darstellt,
- R₁ und R₂ unabhängig voneinander aus der Gruppe H, Methyl gewählt werden daß aber nicht beide Reste gleichzeitig Methyl darstellen,
- R₃ gewählt wird aus der Gruppe H, sowie der verzweigten und unverzweigten, gesättigten und ungesättigten Alkyl- und Acylreste mit 1 - 20 Kohlenstoffatomen.

Die Strukturformel ist nicht so zu interpretieren, daß durch den Index a alle in der Klammer repräsentierten Reste R₁, R₂ bzw R₃ im gesamten Molekül jeweils gleich sein müssen. Vielmehr können diese Reste in jedem der a Fragmente 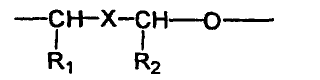 frei gewählt werden

Die Reste A und A' werden vorteilhaft gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema 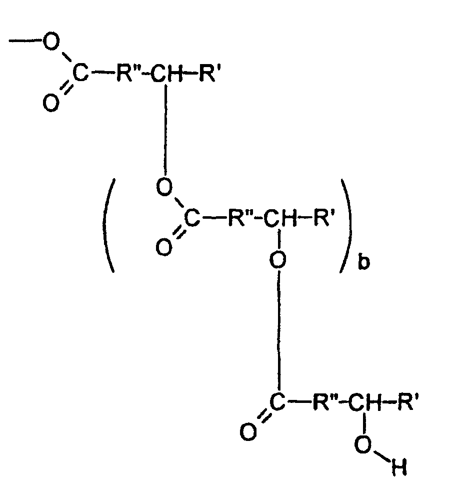 wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann.

Beispiele für besonders vorteilhaft im Sinne der vorliegenden Erfindung zu verwendende W/O-Emulgatoren des A-B-A'-Typs sind PEG-30-Dipolyhydrnxystearat, Decaglycerylheptaoleat, Polyglyceryl-3-Diisostearat, PEG-8-Distearat, Diglycerindipolyhydroxystearat.

Erfindungsgemäß können der oder die W/O-Emulgatoren allerdings auch gewählt werden aus der Gruppe Fettalkohole mit 8 - 30 Kohlenstoffatomen, Monoglycerinester gesättigter und/oder ungesättigter, verzweigter und/oder unverzweigter Alkancarbonsäuren einer Kettenlänge von 8 - 24, insbesondere 12 - 18 C-Atomen, Diglycerinester gesättigter und/oder ungesättigter, verzweigter und/oder unverzweigter Alkancarbonsäuren einer Kettenlänge von 8 - 24, insbesondere 12 - 18 C-Atomen, Triglycerinester gesättigter und/oder ungesättigter, verzweigter und/oder unverzweigter Alkancarbonsäuren einer Kettenlänge von 8 - 24, insbesondere 12 - 18 C-Atomen, Polyglycerinester gesättigter und/oder ungesättigter, verzweigter und/oder unverzweigter Alkancarbonsäuren einer Kettenlänge von 8 - 24, insbesondere 12 - 18 C-Atomen mit bis zu 10 Glycerineinheiten, Monoglycerinether gesättigter und/oder ungesättigter, verzweigter und/oder unverzweigter Alkohole einer Kettenlänge von 8 - 24, insbesondere 12 - 18 C-Atomen, Diglycerinether gesättigter und/oder ungesättigter, verzweigter und/oder unverzweigter Alkohole einer Kettenlänge von 8 - 24, insbesondere 12 - 18 C-Atomen, Triglycerinether gesättigter und/oder ungesättigter, verzweigter und/oder unverzweigter Alkohole einer Kettenlänge von 8 - 24, insbesondere 12 - 18 C-Atomen, Polyglycerinether gesättigter und/oder ungesättigter, verzweigter und/oder unverzweigter Alkancarbonsäuren einer Kettenlänge von 8 - 24, insbesondere 12 - 18 C-Atomen mit bis zu 10 Glycerineinheiten, Propylenglycolester gesättigter und/oder ungesättigter, verzweigter und/oder unverzweigter Alkancarbonsäuren einer Kettenlänge von 8 - 24, insbesondere 12 - 18 C-Atomen, Sorbitanester gesättigter und/oder ungesättigter, verzweigter und/oder unverzweigter Alkancarbonsäuren einer Kettenlänge von 8 - 24, insbesondere 12 - 18 C-Atomen, Sorbitanester von Polyolen, insbesondere des Glycerins, Pentaerythritylester gesättigter und/oder ungesättigter, verzweigter und/oder unverzweigter Alkancarbonsäuren einer Kettenlänge von 8 - 24, insbesondere 12 - 18 C-Atomen, Methylglucose Ester gesättigter und/oder ungesättigter, verzweigter und/oder unverzweigter Alkancarbonsäuren einer Kettenlänge von 8 - 24, insbesondere 12 - 18 C-Atomen, Polyglycerin Methylglucose Ester gesättigter und/oder ungesättigter, verzweigter und/oder unverzweigter Alkancarbonsäuren einer Kettenlänge von 8 - 24, insbesondere 12 -18 C-Atomen.

Es kann erfindungsgemäß von Vorteil sein, daß die vorstehend genannten Typen von W/O-Emulgatoren zusätzlich polyethoxyliert und/oder polypropoxyliert sind, oder daß auch andere polyethoxylierte und/oder polypropoxylierte Produkte Verwendung finden, beispielsweise polyethoxyliertes hydrogeniertes oder nichthydrogeniertes Ricinusöl, ethoxyliertes Cholesterin.

Insbesondere vorteilhafte W/O-Emulgatoren sind Glyceryllanolat, Glycerylmonostearat, Glycerylmonoisostearat, Glycerylmonomyristat, Glycerylmonooleat, Diglycerylmonostearat, Diglycerylmonoisostearat, Diglyceryldiisostearat, Propylenglycolmonostearat, Propylenglycolmonoisostearat, Propylenglycoldiisostearat, Propylenglycolmonocaprylat, Propylenglycolmonolaurat, Sorbitanmanoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, 2-Ethylhexylglycerinether, Selachylalkohol, Chimylalkohol, Polyethylenglycol(2)stearylether (Steareth-2), Glycerylmonolaurat, Glycerylmonocaprinat, Glycerylmonocaprylat, Glycerylsorbitanstearat Polyglyceryl-4 Isostearat, Polyglyceryl-2-sesquiisostearat, PEG-7 hydrogeniertes Castoröl, PEG-40-Sorbitanperisostearat, Isostearyldiglycerylsuccinat, PEG-5-Cholesterylether.

Der erfindungsgemäß verwendete W/O-Emulgator bzw. die erfindungsgemäß verwendeten W/O-Emulgatoren, welcher oder welche in das Schema A-B-A' passen, liegt bzw. liegen vorteilhaft in Konzentrationen von 0,1 - 25 Gew.-% vor, wobei es allerdings möglich und vorteilhaft ist, den Gehalt an Emulgatoren niedrig zu hatten, etwa bis 5 Gew.-%, jeweils bezogen auf das Gesamtgewicht der Zusammensetzung. Es ist vorteilhaft, die Gesamtkonzentration der W/O-Emulgatoren, was auch diejenigen Emulgatoren einschließt, die nicht in das Schema A-B-A' passen, nicht größer als etwa 25 - 30 Gew.-% und nicht geringer als etwa 0,1 Gew.-% zu wählen, jeweils bezogen auf das Gesamtgewicht der Zubereitungen.

Die erfindungsgemäß verwendeten Stabilisatoren werden erfindungsgemäß vorteilhaft gewählt aus der Gruppe der Substanzen der allgemeinen Formel 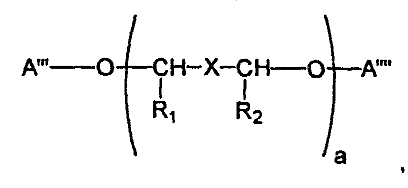 wobei
- A"' und A"" gleiche oder verschiedene hydrophobe organische Reste darstellen,
- a eine Zahl von 1 bis 100, vorzugsweise 2 bis 60, darstellt,
- X eine Einfachbindung oder die Gruppe 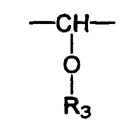 darstellt,
- R₁ und R₂ unabhängig voneinander aus der Gruppe H, Methyl gewählt werden daß aber nicht beide Reste gleichzeitig Methyl darstellen,
- R₃ gewählt wird aus der Gruppe H, sowie der verzweigten und unverzweigten, gesättigten und ungesättigten Alkyl- und Acylreste mit 1 - 20 Kohlenstoffatomen.

Die Stcukturformel ist nicht so zu interpretieren, daß durch den Index a alle in der Klammer repräsentierten Reste R₁, R₂ bzw R₃ im gesamten Molekül jeweils gleich sein müssen. Vielmehr können diese Reste in jedem der a Fragmente 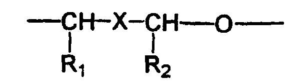 frei gewählt werden.

Die Reste A"' und A"" können gleich oder verschieden sein und werden bevorzugt gewählt aus der Gruppe 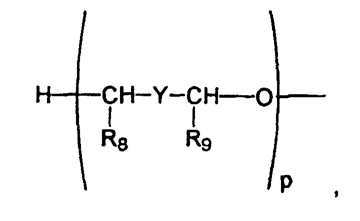 wobei R₈ und R₉ gleich oder verschieden sein können und gewählt werden aus der Gruppe der gesättigten und ungesättigten Alkyl- und Acylreste mit 1 - 30 Kohlenstoffatomen, p eine Zahl von 1 - 20 darstellt und Y eine Einfachbindung oder die Gruppe 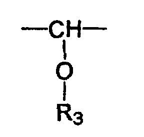 darstellt, wobei R₃ gewählt wird aus der Gruppe H, sowie der verzweigten und unverzweigten, gesättigten und ungesättigten Alkyl- und Acylreste mit 1 - 30 Kohlenstoffatomen.

Bevorzugter Stabilisator ist das PEG-45 /Dodecylglycolcopolymer, welches die Struktur 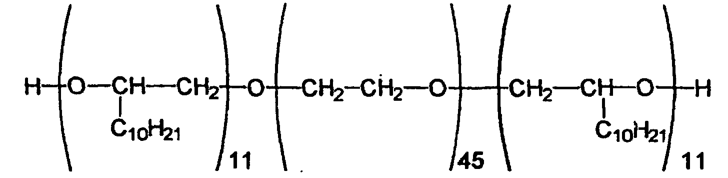 besitzt. Es wird von der Gesellschaft Akzo Nobel Chemicals GmbH unter der Bezeichnung ELFACOS® ST 9 angeboten. Aber auch das entsprechende PEG-22 / Dodecylglycolcopolymer ist vorteilhaft zu verwenden.

Ferner können die Gruppe A"' und A""unabhängig voneinander auch Alkylreste oder Acylreste darstellen. Besonders vorteilhaft ist auch als Stabilisator das Methoxy-PEG-22-Dodecyl Glycol Copolymer zu verwenden. Es wird von der Gesellschaft Akzo Nobel Chemicals GmbH unter der Bezeichnung ELFACOS® E 200 angeboten.

Der Stabilisator bzw. die Stabilisatoren liegen vorteilhaft in Konzentrationen von 0,01 - 25 Gew.-% vor, wobei es allerdings möglich und vorteilhaft ist, den Gehalt an Stabilisatoren niedrig zu halten, etwa bis 5 Gew.-%, jeweils bezogen auf das Gesamtgewicht der Zusammensetzung.

Es ist insbesondere dann vorteilhaft, Stabilisatoren zu wählen, wenn erfindungsgemäße Zubereitungen einen hohen Gehalt an destabilisierenden Substanzen, beispiels-weise Lichtschutzfilter enthalten sollen. Ist der Gehalt an destabilisierenden Substanzen gering, kann man auf den Stabilisator verzichten.

Die Ölkomponente oder die Gesamtheit der Ölkomponenten der erfindungsgemäßen wasserhaltigen, kosmetischen Stifte sollen bei Raumtemperatur eine Flüssigkeit darstellen, die Wachskomponente oder die Gesamtheit der Wachskomponente sollen bei Raumtemperatur einen Festkörper bilden. Es ist von Vorteil, die Ölkomponenten und die Wachskomponenten so aufeinander abzustimmen, daß das Gemisch aus Ölkomponenten und Wachskomponenten ohne restliche Komponenten, also etwa ohne Wasserphase und ohne Emulgator, bei Raumtemperatur einen Festkörper bildet.

Die Ölkomponente oder die Gesamtheit der Ölkomponenten der erfindungsgemäßen wasserhaltigen, kosmetischen Stifte wird bevorzugt gewählt aus der Gruppe der Ester aus gesättigten und/oder ungesättigten, verzweigten und/oder unverzweigten Alkancarbonsäuren einer Kettenlänge von 1 bis 44 C-Atomen und gesättigten und/oder ungesättigten, verzweigten und/oder unverzweigten Alkoholen einer Kettenlänge von 1 bis 44 C-Atomen, aus der Gruppe der Ester aus aromatischen Carbonsäuren und gesättigten und/oder ungesättigten, verzweigten und/oder unverzweigten Alkoholen einer Kettenlänge von 1 bis 30 C-Atomen sofern die Ölkomponente oder die Gesamtheit der Ölkomponenten bei Raumtemperatur eine Flüssigkeit darstellen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe, der Silkonöle, der Dialkylether, der Gruppe der gesättigten oder ungesättigten, verzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkemöl und dergleichen mehr.

Vorteilhaft wird die Ölphase ferner gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether, Ethylenglycoldioleat, Di-(2-Ethylhexyl)adipat).

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Bevorzugt ist, die Ölkomponenten zu wählen aus der Gruppe der
- Ester aus gesättigten verzweigten Alkancarbonsäuren einer Kettenlänge von 1 bis 44 C-Atomen und gesättigten unverzweigten Alkoholen einer Kettenlänge von 1 bis 44 C-Atomen, sofern die Ölkomponente oder die Gesamtheit der Ölkomponenten bei Raumtemperatur eine Flüssigkeit darstellen,
   und/oder der
- Ester aus gesättigten unverzweigten Alkancarbonsäuren einer Kettenlänge von 1 bis 44 C-Atomen und gesättigten verzweigten Alkoholen einer Kettenlänge von 1 bis 44 C-Atomen, sofern die Ölkomponente oder die Gesamtheit der Ölkomponenten bei Raumtemperatur eine Flüssigkeit darstellen.

Besonders bevorzugt ist, die Ölkomponenten zu wählen aus der Gruppe der
- Ester aus gesättigten verzweigten Alkancarbonsäuren einer Kettenlänge von 14 bis 44 C-Atomen und gesättigten verzweigten Alkoholen einer Kettenlänge von 14 bis 44 C-Atomen, sofern die Ölkomponente oder die Gesamtheit der Ölkomponenten bei Raumtemperatur eine Flüssigkeit darstellen.

Besonders vorteilhafte Ölkomponenten können aus der Gruppe Isotridecylisononanoat, Isocetylstearat. Isopropylsiearat, Isopropylisostearat, Butyloctansäure-2-butyloctanoat, 2-Ethylhexylisostearat (=Octylisostearat) Cetearylisononanoat, C₁₂-C₁₅-Alkylbenzoat, C₁₂-C₁₅-Alkohollactat, Glyceryltriisostearin, Cyclomethicon, Isohexadecan, gewählt werden.

Die Ölkomponenten können vorteilhaft in einem Gehalt von 0,5 bis 80 Gew.-%, bezogen auf die Gesamtzubereitung vorliegen, bevorzugt sind etwa 1 bis 20 Gew.-%.

Die Wachskomponente oder die Gesamtheit der Wachskomponenten der erfindungsgemäßen wasserhaltigen, kosmetischen Stifte wird bevorzugt gewählt aus der Gruppe der Ester aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, aus der Gruppe der Ester aus aromatischen Carbonsäuren bzw. Hydroxycarbonsäuren (z.B. 12-Hydroxystearinsäure) und gesättigten und/oder ungesättigten, verzweigten und/oder unverzweigten Alkoholen einer Kettenlänge von 1 bis 80 C-Atomen sofern die Wachskomponente oder die Gesamtheit der Wachskomponenten bei Raumtemperatur einen Festkörper darstellen.

Bevorzugt ist, die Wachskomponenten zu wählen aus der Gruppe der
- Ester aus gesättigten verzweigten Alkancarbonsäuren einer Kettenlänge von 1 bis 60 C-Atomen und gesättigten unverzweigten Alkoholen einer Kettenlänge von 1 bis 60 C-Atomen, sofern die Wachskomponente oder die Gesamtheit der Wachskomponenten bei Raumtemperatur einen Festkörper darstellen,
   und/oder der
- Ester aus gesättigten unverzweigten Alkancarbonsäuren einer Kettenlänge von 1 bis 60 C-Atomen und gesättigten verzweigten Alkoholen einer Kettenlänge von 1 bis 60 C-Atomen, sofern die Wachskomponente oder die Gesamtheit der Wachskomponenten bei Raumtemperatur einen Festkörper darstellen.

Besonders bevorzugt ist, die Wachskomponenten zu wählen aus der Gruppe der
- Ester aus gesättigten verzweigten Alkancarbonsäuren einer Kettenlänge von 14 bis 44 C-Atomen und gesättigten verzweigten Alkoholen einer Kettenlänge von 14 bis 44 C-Atomen, sofern die Wachskomponente oder die Gesamtheit der Wachskomponenten bei Raumtemperatur einen Festkörper darstellen.

Insbesondere vorteilhaft können die Wachskomponenten aus der Gruppe der C₁₆₋₃₆-Alkylstearate, der C₁₀₋₄₀-Alkylstearate, der C₂₀₋₄₀Alkylisostearate, der C₂₀₋₄₀-Dialkyldimerate, der C₁₈₋₃₈-Alkylhydroxystearoylstearate, der C₂₀₋₄₀-Alkylerucate gewählt werden, ferner C₃₀₋₅₀-Alkylbienenwachs, Cetearylbehenat. Auch Siliconwachse wie beispielsweise Stearyltrimethylsilan/Stearylalkohol sind gegebenenfalls vorteilhaft.

Insbesondere pflanzliche und/oder tierische Wachse oder chemisch modifizierte Derivate davon, insbesondere Camaubawachs, Candelillawachs, Sonnenblumenwachs, Reiswachse, Fruchtwachse wie Orangenwachs, Zitronenwachs, Grapefruitwachs, Lorbeerwachs (= Bayberrywax) und dergleichen, sind vorteilhaft zu verwenden. Außerdem können diese natürlichen Wachse auch ohne synthetische Wachse allein eingesetzt werden.

Die Wachskomponenten können vorteilhaft in einem Gehalt von 0,5 bis 80 Gew.-%, bezogen auf die Gesamtzubereitung vorliegen, bevorzugt sind etwa 1 bis 20 Gew.-%.

Es ist von Vorteil, das Verhältnis von Öl- und Wachskomponenten zueinander ungefähr aus dem Bereich der Gewichtsverhältnisse zwischen 2 : 1 bis 1 : 2, insbesondere 3 : 2 bis 2 : 3, ganz besonders bevorzugt ca. 1 : 1, einzustellen.

Die Wassermenge kann bis zu etwa 85 Gew.-% betragen, bezogen auf das Gesamtgewicht der Zubereitungen, wobei üblicherweise optimale Wassergehalte im Bereich zwischen 50 und 75 Gew.-% gewählt werden.

Es ist auch möglich, zusätzliche Substanzen zu verwenden, welche die Konsistenz der erfindungsgemäßen Zubereitungen modifizieren, beispielsweise Verdicker, welche gewählt werden können aus der Gruppe der Substanzen welche mindestens zwei hydrophile Reste tragen, welche über eine hydrophobe Gruppierung miteinander verbunden sind, also den Molekülschemata B'―A"―B" 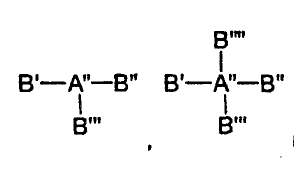 usw.
folgen.
Dabei stellen die Reste B mit den verschiedenen Indizes hydrophile Gruppen dar, die Reste A mit den verschiedenen Indizes hydrophobe Gruppen.

Solche Verdicker werden bevorzugt gewählt aus der Gruppe der Triblockcopolymere des Typs 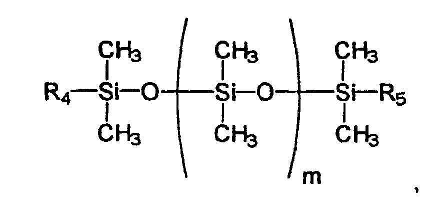 wobei m eine Zahl von 10 bis 10000 darstellen kann, R₄ und R₅ gleich oder verschieden sein können und gewählt werden aus der Gruppe, die durch die allgemeine Struktur 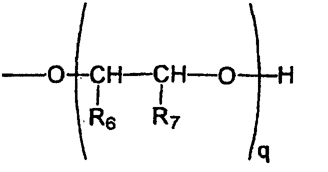 repräsentiert wird. Dabei können R₆ und R₇ unabhängig voneinander so gewählt werden daß sie H und Methyl, daß aber nicht beide Reste gleichzeitig Methyl darstellen können. q ist eine Zahl von 2 bis 1000, bevorzugt von 10 bis 200.

R₄ und R₅ können auch Polylolreste darstellen (z.B. Glyceryl-, Polyglyceryl-, Sorbityl-, Cellulosereste usw.).

Der Stabilisator bzw. die Stabilisatoren liegen vorteilhaft in Konzentrationen von 0,01 - 25 Gew.-% vor, wobei es allerdings möglich und vorteilhaft ist, den Gehalt an Stabilisatoren niedrig zu halten, etwa bis 5 Gew.-%, jeweils bezogen auf das Gesamtgewicht der Zusammensetzung.

In die erfindungsgemäßen Zubereitungen können vorteilhaft zusätzlich die üblichen Bestandteile kosmetischer Stifte eingearbeitet werden, z.B. Kohlenwasserstoffe, Fette und Öle für die Grundsubstanz, sowie die üblichen Hilfs- und Zusatzstoffe wie Parfümöle, Konservierungsmittel, Farbpigmente, Lichtschutzmittel, Stabilisatoren.

Insbesondere dann, wenn die erfindungsgemäßen Zubereitungen sich durch leichte oder erleichterte Abwaschbarkeit von menschlicher Haut auszeichnen sollen, ist es von Vorteil, den Zubereitungen wasserlösliche und/oder mit Wasser quellbare Polymere einzuverleiben, insbesondere mit Alkylgruppen veretherte Cellulose- und/oder Stärkederivate. Besonders vorteilhaft sind β-Glucane, Xanthangummi, Dextrane, Hydroxymethylcellulose, Hydroxyethylcellulose und/oder Hydroxypropylcellulose, Methoxy-PEG-22/Dodecyl-Glycol-Copolymere, Poloxamere.

Vorteilhafte wasserlösliche und/oder mit Wasser quellbare Polymere können auch gewählt werden als mit einem oder mehreren n-Octenylsuccinatresten veresterter hydrophiler Stärke enthalten. Solche Stärkederivate zeichnen sich aus durch eine Struktur

Stärke-Xₙ, wobei X den Rest 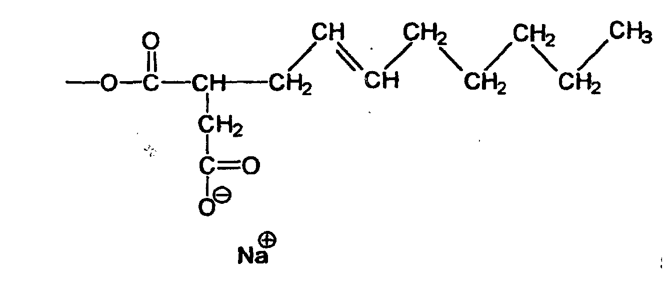 symbolisiert.

Erfindungsgemäß vorteilhaft zu verwendende Stärkederivate tragen offiziell noch keinen INCI-Namen (International Nomenclature Cosmetic Ingredient) dieser müßte die Bezeichnung "Starch Sodium Octenyl Succinate" tragen. Besonders vorteilhaft sind solche Produkte, welcher unter der Bezeichnung Amiogum®, insbesondere Amiogum®23 von der Gesellschaft Cerestar US verkauft werden.

Es wird bevorzugt, den Gehalt an wasserlöslichen und/oder mit Wasser quellbaren Polymeren im Konzentrationsbereich von 0,01 - 5,0 Gew.-%, besonders bevorzugt 0,1 - 1,0 Gew.-%, zu wählen.

Es, hat sich in erstaunlicher Weise herausgestellt, daß die erfindungsgemäß verwendeten wasserlöslichen und/oder mit Wasser quellbaren Polymere darüberhinaus die Hautfreundlichkeit der erfindungsgemäßen kosmetischen Zubereitungen erhöhen. Es wird ein angenehmeres Gefühl beim Auftragen der Stiftmasse auf die Haut erzielt.

Die Einarbeitung solcher wasserlöslichen und/oder mit Wasser quellbaren Polymere erfolgt bevorzugt dadurch, daß sie der Wasserphase einverleibt und mit der Wasserphase, besonders bevorzugt nach vollständiger Auflösung bzw. Quellung in die aufgeschmolzene Fettphase der Zubereitungen gegeben werden.

Zusätzlich können Pflegewirkstoffe eingearbeitet werden, welche sich nicht wie bisher auf die fettlöslichen Wirkstoffe beschränken, sondern auch aus der Gruppe der wasserlöslichen Wirkstoffe gewählt werden können, beispielsweise Vitamine und dergleichen mehr.

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch, beispielsweise zum Schutze der Formulierung selbst, einen Gehalt an UV-Schutzsubstanzen enthalten. So werden z.B. in Tagescremes gewöhnlich UV-A- bzw. UV-B-Filtersubstanzen eingearbeitet.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische und/oder dermatologische Zubereitungen zur Verfügung zu stellen, die die Haut vor dem gesamten Bereich der ultravioletten Strahlung schützen. Sie können auch als Sonnenschutzmittel dienen.

Die UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher,
- 4-Aminobenzoesäure-Derivate, vorzugsweise 4-(Dimethylamino)-benzoesäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester,
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin

Als wasserlösliche Substanzen sind vorteilhaft:
- 2-Phenylbenzimidazol-5-suifonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Substanzen handelt es sich vorzugsweise um Derivate des Dibenzoylmethans, insbesondere um 1-(4'-tert.Butytphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, weiche für UVB-Filtersubstanzen genannt wurden.

Erfindungsgemäße kosmetische und/oder dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Die erfindungsgemäßen wasserreichen Stifte sind ferner hervorragende Vehikel für dermatologische Wirkstoffe. Insbesondere eignen sie sich als Träger für gegen Akne wirksame Substanzen. So ist es vorteilhaft, den erfindungsgemäß verwendeten Zubereitungen gegen Akne wirksame Substanzen zuzugeben, beispielsweise gegen *Propionibacterium acnes* wirksame Stoffe (etwa solche, die in DE-OS 42 29 707, DE-OS 43 05 069, DE-OS 43 07 976, DE-OS 43 37 711, DE-OS 43 29 379 beschrieben werden) aber auch andere gegen Akne wirksame Substanzen, beispielsweise all-trans-Retinsäure, 13-cis-Retinsäure und verwandte Stoffe) oder antientzündliche Wirkstoffe, beispielsweise Batylalkohol (α-Octadecylglycerylether), Selachylalkohol (α-9-Octadecenylglycerylether), Chimylalkohol (α-Hexadecylglycerylether) und/oder Bisabolol.

Die Menge der Antiaknemittel (eine oder mehrere Verbindungen) in den Zubereitungen beträgt vorzugsweise 0,01 bis 30 Gew.-%, besonders bevorzugt 0,1 - 20 Gew.-%, insbesondere 1 - 10 Gew.-%, bezogen auf das Gesamtgewicht der Zubereitung.

Vorteilhafte Wirkstoffe sind weiterhin Antioxidantien, insbesondere solche, welche nicht nur die Bestandteile der Formulierung, sondern auch die Haut vor oxidativer Beanspruchung schützen können.

Die Zubereitungen enthalten daher vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden. Es ist dabei vorteilhaft, Antioxidantien als einzige Wirkstoffklasse zu verwenden, etwa dann, wenn eine kosmetische oder dermatologische Anwendung im Vordergrunde steht wie die Bekämpfung der oxidativen Beanspruchung der Haut. Es ist aber auch günstig, die erfindungsgemäßen W/O-Emulsionsstifte mit einem Gehalt an einem oder mehreren Antioxidantien zu versehen, wenn die Zubereitungen einem anderen Zwecke dienen sollen, z.B. als Desodorantien oder Sonnenschutzmittel.

Besonders vorteilhaft werden die Antioxidantien gewählt aus der Gruppe, bestehend aus Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothiogtucose, Propylthlouracil und andere Thiole (z.B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Pentat-, Hexa-, Heptahioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol deren Derivate, Vitamin C und Derivate (z.B. Ascorbylpalmitate, Mg - Ascorbylphosphate, Ascorbylacetate), Isoascorbinsäure und ihre Derivate, Tocopherole und Derivate (z.B. Vitamin E - acetat), Vitamin A und Derivate (Vitamin A - palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können zwar öllösliche oder öldispergierbare Antioxidantien eingesetzt werden. Es hat sich jedoch herausgestellt, daß die Erfindung gerade dem Einsatz wasserlöslicher oder wasserdispergierbarer Antioxidantien in Stiftformulierungen die Pforten öffnet.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist es vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmanne natürlich bekannt, daß anspruchsvolle kosmetische Zubereitungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Erfindungsgemäß können Wirkstoffe auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die γ-Linoiensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkemöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl®, Eucerit® und Neocerit®.

Die erfindungsgemäßen Stifte tragen ferner in vorzüglicher Weise zur Hautglättung bei, insbesondere, wenn sie mit einer oder mehreren Substanzen versehen sind, die die Hautglättung fördern.

Es ist gegebenenfalls möglich und vorteilhaft, die erfindungsgemäßen Zubereitungen als Grundlage für pharmazeutische Formulierungen zu verwenden. Mutatis mutandis gelten entsprechende Anforderungen an die Formulierung medizinischer Zubereitungen. Die Übergänge zwischen reinen Kosmetika und reinen Pharmaka sind dabei fließend. Als pharmazeutische Wirkstoffe sind erfindungsgemäß grundsätzlich alle Wirkstoffklassen geeginet, wobei lipophile Wirkstoffe bevorzugt sind. Beispiele sind: Antihistaminika, Antiphlogistika, Antibiotika, Antimykotika, die Durchblutung fördernde Wirkstoffe, Keratolytika, Hormone, Steroide, Vitamine usw.

Alle Mengenangaben, Prozentangaben oder Teile beziehen sich, soweit nicht anders angegeben, auf das Gewicht, insbesondere auf das Gesamtgewicht der Zubereitungen oder der jeweiligen Mischungen.

Die nachfolgenden Beispiele sollen die Erfindung erläutern, ohne sie einzuschränken.

### Beispiel 1

| Sonnenschutzstift mit hohem Wasseranteil | |
|---|---|
| | Gew.-% |
| Polyglyceryl-3-diisostearat | 1,800 |
| Caprylic/Capric Triglyceride | 5,000 |
| Octyldodecanol | 5,000 |
| Dicaprylylether | 5,000 |
| Butylmethoxydibenzoylmethan | 2,000 |
| Phenylbenzimidazolsulfonsäure | 4,000 |
| Natronlauge | 0.200 |
| Titandioxid | 2.000 |
| C₂₀₋₄₀-Alkylstearat | 15,000 |
| Glycerin | 2,000 |
| Na₃HEDTA | 2,000 |
| Wasser | ad 100,000 |

### Beispiel 2

| Sonnenschutzstift mit hohem Wasseranteil | |
|---|---|
| | Gew.-% |
| PEG-45/ Dodecyl Glycol Copolymer | 0,900 |
| Polyglyceryl-3-diisostearat | 1,800 |
| Caprylic/Capric Triglyceride | 5,000 |
| Octyldodecanol | 5,000 |
| Dicaprylylether | 5,000 |
| Butylmethoxydibenzoylrnethan | 2,000 |
| Methylbenzylidencampher | 4,000 |
| Titandioxid | 2.000 |
| C₂₀₋₄₀-Alkylstearat | 15,000 |
| Glycerin | 2,000 |
| Na₃HEDTA | 2,000 |
| Wasser | ad 100,000 |

### Beispiel 3

| Sonnenschutzstift mit hohem Wasseranteil | |
|---|---|
| | Gew.-% |
| PEG-45/ Dodecyl Glycol Copolymer | 0,900 |
| Polyglyceryl-3-diisostearat | 1,800 |
| Caprylic/Capric Triglyceride | 5,000 |
| Octyldodecanol | 5,000 |
| Dicaprylylether | 5,000 |
| Butyimethoxydibenzoylmethan | 2,000 |
| Phenylbenzimidazolsulfonsäure | 4,000 |
| Titandioxid | 2.000 |
| Natronlauge | 0.200 |
| C₂₀₋₄₀-Alkylstearat | 15,000 |
| Glycerin | 2,000 |
| Na₃HEDTA | 2,000 |
| Wasser | ad 100,000 |

### Beispiel 4

| Sonnenschutzstift mit hohem Wasseranteil | |
|---|---|
| | Gew.-% |
| PEG-45/ Dodecyl Glycol Copolymer | 0,900 |
| Polyglyceryl-3-diisostearat | 1,800 |
| Caprylic/Capric Triglyceride | 5,000 |
| Octyldodecanol | 5,000 |
| Dicaprylylether | 5,000 |
| Butylmethoxydibenzoylmethan | 2,000 |
| Methylbenzylidencampher | 4,000 |
| Titandioxid* und Proylenglycol | 2.000 |
| Natronlauge | 0.200 |
| C₂₀₋₄₀-Alkylstearat | 15,000 |
| Glycerin | 2,000 |
| Wasser | ad 100,000 |

| | |
|---|---|
| * Wasserdispergierbares Titandioxid: Tioveil AQ+10%Propylenglycol von Tioxid Specalities | |

### Beispiel 5

| Sonnenschutzstift mit hohem Wasseranteil | |
|---|---|
| | Gew.-% |
| PEG-45/ Dodecyl Glycol Copolymer | 0,900 |
| 2-Ethylhexylgtycerinether | 1,800 |
| Caprylic/Capric Triglyceride | 5,000 |
| Octyldodecanol | 5,000 |
| Dicaprylylether | 5,000 |
| Butylmethoxydibenzoylmethan | 2,000 |
| Methylbenzylidencampher | 4,000 |
| Titandioxid* und Proylenglycol | 2,000 |
| Natronlauge | 0,200 |
| C₂₀₋₄₀-Alkylstearat | 15,000 |
| Glycerin | 2,000 |
| Na₃HEDTA | 2,000 |
| Wasser | ad 100,000 |

| | |
|---|---|
| * Wasserdispergierbares Titandioxid: Tioveil AQ+10%Propylenglycol von Tioxid Specalities | |

### Beispiel 7

| Sonnenschutzstift mit hohem Wasseranteil | |
|---|---|
| | Gew.-% |
| PEG-45/ Dodecyl Glycol Copolymer | 0.900 |
| Sorbitanisostearat | 1,800 |
| Caprylic/Capric Triglyceride | 5,000 |
| Octyldodecanol | 5,000 |
| Dicaprylylether | 5,000 |
| Butylmethoxydibenzoylmethan | 2,000 |
| Phenylbenzimidazolsulfonsäure | 4,000 |
| Natronlauge | 0,200 |
| Titandioxid | 2,000 |
| C₂₀₋₄₀-Alkylstearat | 15,000 |
| Glycerin | 2,000 |
| Na₃HEDTA | 2,000 |
| Wasser | ad 100,000 |

### Beispiel 8

| Sonnenschutzstift mit hohem Wasseranteil | |
|---|---|
| | Gew.-% |
| PEG-45/ Dodecyl Glycol Copolymer | 0.900 |
| Pentaerythritylisostearat | 1,800 |
| Caprylic/Capric Triglyceride | 5,000 |
| Octyldodecanol | 5,000 |
| Dicaprylylether | 5,000 |
| Butylmethoxydibenzoylmethan | 2,000 |
| Phenylbenzimidazolsulfonsäure | 4,000 |
| Natronlauge | 0,200 |
| Titandioxid | 2,000 |
| C₂₀₋₄₀-Alkylstearat | 15,000 |
| Glycerin | 2,000 |
| Na₃HEDTA | 2,000 |
| Wasser | ad 100,000 |

### Beispiel 9

| Sonnenschutzstift mit hohem Wasseranteil | |
|---|---|
| | Gew.-% |
| PEG-45/ Dodecyl Glycol Copolymer | 0.900 |
| PEG- 7 hydrogeniertes Ricinusöl | 1,800 |
| Caprylic/Capric Triglyceride | 5,000 |
| Octyldodecanol | 5,000 |
| Dicaprylylether | 5,000 |
| Butylmethoxydibenzoylmethan | 2,000 |
| Phenylbenzimidazolsulfonsäure | 4,000 |
| Natronlauge | 0,200 |
| Titandioxid | 2,000 |
| C₂₀₋₄₀-Alkylstearat | 15,000 |
| Glycerin | 2,000 |
| Na₃HEDTA | 2,000 |
| Wasser | ad 100,000 |

### Beispiel 10

| Sonnenschutzstift mit hohem Wasseranteil | |
|---|---|
| | Gew.-% |
| PEG-45/ Dodecyl Glycol Copolymer | 0.900 |
| Polyglyceryl-2 Dipolyhydroxystearat | 1,800 |
| Caprylic/Capric Triglyceride | 5,000 |
| Octyldodecanol | 5,000 |
| Dicaprylylether | 5,000 |
| Butylmethoxydibenzoylmethan | 2,000 |
| Phenylbenzimidazolsulfonsäure | 4,000 |
| Natronlauge | 0,200 |
| Titandioxid | 2,000 |
| C₂₀₋₄₀-Alkylstearat | 15,000 |
| Glycerin | 2,000 |
| Na₃HEDTA | 2,000 |
| Wasser | ad 100,000 |

### Beispiel 11

| Sonnenschutzstift mit hohem Wasseranteil | |
|---|---|
| | Gew.-% |
| PEG-45/ Dodecyl Glycol Copolymer | 0,900 |
| PEG-40 Sorbitanperisostearat | 1,800 |
| Caprylic/Capric Triglyceride | 5,000 |
| Octyldodecanol | 5,000 |
| Dicaprylylether | 5,000 |
| Butylmethoxydibenzoylmethan | 2,000 |
| Phenylbenzimidazolsulfonsäure | 4,000 |
| Natronlauge | 0,200 |
| Titandioxid | 2,000 |
| C₂₀₋₄₀-Alkylstearat | 15,000 |
| Glycerin | 2,000 |
| Na₃HEDTA | 2,000 |
| Wasser | ad 100,000 |

### Beispiel 12

| Sonnenschutzstift mit hohem Wasseranteil | |
|---|---|
| | Gew.-% |
| PEG-45/ Dodecyl Glycol Copolymer | 0,900 |
| Isostearyl Diglycerin Succinat | 1,800 |
| Caprylic/Capric Triglyceride | 5,000 |
| Octyldodecanol | 5,000 |
| Dicaprylylether | 5,000 |
| Butylmethoxydibenzoylmethan | 2,000 |
| Phenylbenzimidazolsulfonsäure | 4,000 |
| Natronlauge | 0,200 |
| Titandioxid | 2,000 |
| C₂₀₋₄₀-Alkylstearat | 15,000 |
| Glycerin | 2,000 |
| Na₃HEDTA | 2,000 |
| Wasser | ad 100,000 |

### Beispiel 13

| Sonnenschutzstift mit hohem Wasseranteil | |
|---|---|
| | Gew.-% |
| PEG-45/ Dodecyl Glycol Copolymer | 0,900 |
| Glycerinisostearat | 1,800 |
| Caprylic/Capric Triglyceride | 5,000 |
| Octyldodecanol | 5,000 |
| Dicaprylylether | 5,000 |
| Butylmethoxydibenzoylmethan | 2,000 |
| Phenylbenzimidazolsulfonsäure | 4,000 |
| Natronlauge | 0,200 |
| Titandioxid | 2,000 |
| C₂₀₋₄₀-Alkylstearat | 15,000 |
| Glycerin | 2,000 |
| Na₃HEDTA | 2,000 |
| Wasser | ad 100,000 |

### Beispiel 14

| Sonnenschutzstift mit hohem Wasseranteil | |
|---|---|
| | Gew.-% |
| PEG-45/ Dodecyl Glycol Copolymer | 0,900 |
| Cetylalkohol | 1,800 |
| Caprylic/Capric Triglyceride | 5,000 |
| Octyldodecanol | 5,000 |
| Dicaprylylether | 5,000 |
| Butylmethoxydibenzoylmethan | 2,000 |
| Phenylbenzimidazolsulfonsäure | 4,000 |
| Natronlauge | 0,200 |
| Titandioxid | 2,000 |
| C₂₀₋₄₀-Alkylstearat | 15,000 |
| Glycerin | 2,000 |
| Na₃HEDTA | 2,000 |
| Wasser | ad 100,000 |

### Beispiel 15

| Sonnenschutzstift mit hohem Wasseranteil | |
|---|---|
| | Gew.-% |
| PEG-45/ Dodecyl Glycol Copolymer | 0,900 |
| Propylenglycoldiisostearat | 1,800 |
| Caprylic/Capric Triglyceride | 5,000 |
| Octyldodecanol | 5,000 |
| Dicaprylylether | 5,000 |
| Butylmethoxydibenzoylmethan | 2,000 |
| Phenylbenzimidazolsulfonsäure | 4,000 |
| Natronlauge | 0,200 |
| Titandioxid | 2,000 |
| C₂₀₋₄₀-Alkylstearat | 15,000 |
| Glycerin | 2,000 |
| Na₃HEDTA | 2,000 |
| Wasser | ad 100,000 |

### Beispiel 16

| Sonnenschutzstift mit hohem Wasseranteil | |
|---|---|
| | Gew.-% |
| PEG-45/ Dodecyl Glycol Copolymer | 0,900 |
| Isostearylglycerinether | 1,800 |
| Caprylic/Capric Triglyceride | 5,000 |
| Octyldodecanol | 5,000 |
| Dicaprylylether | 5,000 |
| Butylmethoxydibenzoylmethan | 2,000 |
| Phenylbenzimidazolsulfonsäure | 4,000 |
| Natronlauge | 0,200 |
| Titandioxid | 2,000 |
| C₂₀₋₄₀-Alkylstearat | 15,000 |
| Glycerin | 2,000 |
| Na₃HEDTA | 2,000 |
| Wasser | ad 100,000 |

### Beispiel 17

| Sonnenschutzstift mit hohem Wasseranteil | |
|---|---|
| | Gew.-% |
| PEG-45/ Dodecyl Glycol Copolymer | 0,900 |
| Glycerin Sorbitan Isostearat | 1,800 |
| Caprylic/Capric Triglyceride | 5,000 |
| Octyldodecanol | 5,000 |
| Dicaprylylether | 5,000 |
| Butylmethoxydibenzoylmethan | 2,000 |
| Phenylbenzimidazolsulfonsäure | 4,000 |
| Natronlauge | 0,200 |
| Titandioxid | 2,000 |
| C₂₀₋₄₀-Alkylstearat | 15,000 |
| Glycerin | 2,000 |
| Na₃HEDTA | 2,000 |
| Wasser | ad 100,000 |

### Beispiel 18

| Antiaknestift mit hohem Wasseranteil | |
|---|---|
| | Gew.-% |
| Polyglyceryl-3 Diisostearat | 1,600 |
| Glycerylcaprate | 1,000 |
| Caprylic/Capric Triglyceride | 4,000 |
| Octyldodecanol | 4,000 |
| Dicaprylylether | 4,000 |
| Cetearylbehenat | 6,000 |
| Salicylsäure | 1,000 |
| Octacosanylstearat | 6,000 |
| Glycerin | 2,000 |
| Wasser | ad 100,000 |

### Beispiel 19

| Antiaknestift mit hohem Wasseranteil | |
|---|---|
| | Gew.-% |
| Potyglyceryl-3 Diisostearat | 1,600 |
| Glycerylcaprat | 1,000 |
| Caprylic/Capric Triglyceride | 4,000 |
| Octyldodecanol | 4,000 |
| Dicaprylylether | 4,000 |
| Cetearylbehenat | 6,000 |
| Salicylsäure | 1,000 |
| Octacosanylstearat | 6,000 |
| Glycerin | 2,000 |
| Wasser | ad 100,000 |

### Beispiel 20

| Antiaknestift mit hohem Wasseranteil | |
|---|---|
| | Gew.-% |
| Polyglyceryl-3 Diisostearat | 1,600 |
| Azelainsäure | 2.000 |
| Caprylic/Capric Triglyceride | 4,000 |
| Octyldodecanol | 4,000 |
| Dicaprylylether | 4,000 |
| Cetearylbehenat | 6,000 |
| Octacosanylstearat | 6,000 |
| Glycerin | 2,000 |
| Wasser | ad 100,000 |

### Beispiel 21

| Antiaknestift mit hohem Wasseranteil | |
|---|---|
| | Gew.-% |
| Polyglyceryl-3 Diisostearat | 1.600 |
| Wollwachssäure | 2.000 |
| Caprylic/Capric Triglyceride | 4,000 |
| Octyldodecanol | 4,000 |
| Dicaprylylether | 4,000 |
| Cetearyl Behenat | 6,000 |
| Octacosanylstearat | 6,000 |
| Wasser | ad 100,000 |

### Beispiel 22

| Antiaknestift mit hohem Wasseranteil | |
|---|---|
| | Gew.-% |
| Polyglyceryl-3 Diisostearat | 1,600 |
| Azelainsäure | 2,000 |
| Caprylic/Capric Triglyceride | 4,000 |
| Octyldodecanol | 4,000 |
| Dicaprylylether | 4,000 |
| Cetearylbehenat | 6,000 |
| Octacosanylstearat | 6,000 |
| Glycerin | 2,000 |
| Wasser | ad 100,000 |

### Beispiel 23

| Antiaknestift mit hohem Wasseranteil | |
|---|---|
| | Gew.-% |
| Polyglyceryl-3 Diisostearat | 1,600 |
| Azelainsäure | 2,000 |
| Caprylic/Capric Triglyceride | 4,000 |
| Octyldodecanol | 4,000 |
| Dicaprylylether | 4,000 |
| Cetearylbehenat | 6,000 |
| Octacosanylstearat | 6,000 |
| Glycerin | 2,000 |
| Wasser | ad 100,000 |

### Beispiel 24

| Antiaknestift mit hohem Wasseranteil | |
|---|---|
| | Gew.-% |
| Polyglyceryl-3 Dlisostearat | 1,600 |
| Benzoylperoxid | 2,000 |
| Caprylic/Capric Triglyceride | 4,000 |
| Octyldodecanol | 4,000 |
| Dicaprylylether | 4,000 |
| Cetearylbehenat | 6,000 |
| Octacosanylstearat | 6,000 |
| Glycerin | 2,000 |
| Wasser | ad 100,000 |

### Beispiel 25

| Lippenstift mit hohem Wasseranteil | |
|---|---|
| | Gew.-% |
| Polyglyceryl-3 Diisostearat | 1,600 |
| Eisenoxid | 2,000 |
| Caprylic/Capric Triglyceride | 4,000 |
| Octyldodecanol | 4,000 |
| Dicaprylylether | 4,000 |
| Cetearylbehenat | 6,000 |
| Titandioxid | 2,000 |
| Octacosanylstearat | 6,000 |
| Glycerin | 2,000 |
| Wasser | ad 100,000 |

### Beispiel 26

| Lippenstift mit hohem Wasseranteil | |
|---|---|
| | Gew.-% |
| Polyglyceryl-3 Diisostearat | 1,600 |
| Cholesterylalkohol | 2,000 |
| Caprylic/Capric Triglyceride | 4,000 |
| Octyldodecanol | 4,000 |
| Dicaprylylether | 4,000 |
| Cetearylbehenat | 6,000 |
| Titandioxid | 2,000 |
| Octacosanylstearat | 6,000 |
| Glycerin | 2,000 |
| Wasser | ad 100,000 |

### Beispiel 27

| Sonnenschutzstift mit hohem Wasseranteil | |
|---|---|
| | Gew.-% |
| Polyglyceryl-3-diisostearat | 1,800 |
| Caprylic/Capric Triglyceride | 5,000 |
| Octyldodecanol | 5,000 |
| Dicaprylylether | 5,000 |
| Butylmethoxydibenzoylmethan | 2,000 |
| Phenylbenzimidazolsulfonsäure | 4,000 |
| Natronlauge | 0,200 |
| Titandioxid | 2,000 |
| C₂₀₋₄₀-Alkylstearat | 13,000 |
| Orangenwachs | 2,000 |
| Glycerin | 2,000 |
| Na₃HEDTA | 2,000 |
| Wasser | ad 100,000 |

## Patentansprüche

1. Kosmetische Stifte, insbesondere solche, gewählt aus der Gruppe der Lippenstifte, Antiaknestifte, Sonnenschutzstifte und Lidschattenstifte, **dadurch gekennzeichnet, dass** sie
(a) eine Fettphase , welche
(a1) mindestens eine Ölkomponente
(a2) mindestens eine Wachskomponente
(a3) gegebenenfalls weitere in der Fettphase lösliche oder dispergierbare Substanzen
umfaßt:
(b) eine Wasserphase, welche
(b1) 50 bis 85 Gew.% Wasser bezogen auf das Gesamtgewicht der Stiftfüllgutmasse sowie
(b2) gewünschtenfalls in Wasser lösliche oder dispergierbare Substanzen umfaßt,
(c) gewünschtenfalls einen oder mehrere Wirkstoffe, gewählt aus der für Lippenstifte, Antiaknestifte, Sonnenschutzstifte und Lidschattenstifte bekannten Wirkstoffe,
(d) mindestens einen W/O-Emulgator oder ein Gemisch aus mehreren W/O-Emulgatoren,
(e) eine oder mehrere Stabilisatoren, gewählt aus der Gruppe der Substanzen der allgemeinen Struktur A-B-A', wobei A und A' gleiche oder verschiedene hydrophobe organische Reste darstellen und B eine hydrophile Gruppe bedeutet,
(f) gewünschtenfalls weitere grenzflächenaktive Substanzen als Coemulgatoren enthalten, ferner gewünschtenfalls weitere Stabilisatoren und weitere übliche kosmetische und/oder pharmazeutische Hilfs-, Wirk- und/oder Zusatzstoffe, enthalten.

2. Verfahren zur Herstellung kosmetischer Stifte, insbesondere solche, gewählt aus der Gruppe der Lippenstifte, Antiaknestifte, Sonnenschutzstifte und Lidschattenstifte, die **dadurch gekennzeichnet sind, dass** sie
(a) eine Fettphase , welche
(a1) mindestens eine Ölkomponente
(a2) mindestens eine Wachskomponente
(a3) gegebenenfalls weitere in der Fettphase lösliche oder dispergierbare Substanzen
umfaßt:
(b) eine Wasserphase, welche
(b1) 30 bis 85 Gew.% Wasser bezogen auf das Gesamtgewicht der Stiftfüllgutmasse sowie
(b2) gewünschtenfalls in Wasser lösliche oder dispergierbare Substanzen umfaßt,
(c) gewünschtenfalls einen oder mehrere Wirkstoffe, gewählt aus der für Lippenstifte, Antiaknestifte, Sonnenschutzstifte und Lidschattenstifte bekannten Wirkstoffe,
(d) mindestens einen W/O-Emulgator oder ein Gemisch aus mehreren W/O-Emulgatoren,
(e) eine oder mehrere Stabilisatoren, gewählt aus der Gruppe der Substanzen der allgemeinen Struktur A-B-A', wobei A und A' gleiche oder verschiedene hydrophobe organische Reste darstellen und B eine hydrophile Gruppe bedeutet,
(f) gewünschtenfalls weitere grenzflächenaktive Substanzen als Coemulgatoren enthalten, ferner gewünschtenfalls weitere Stabilisatoren und weitere übliche kosmetische und/oder pharmazeutische Hilfs-, Wirk- und/oder Zusatzstoffe,
enthalten,
welches **dadurch gekennzeichnet ist, dass** die Wasserphase und die erwärmte Fettphase in einem Ein-Schritt-Verfahren zusammengegeben und anschließend auf Raumtemperatur abgekühlt werden.

3. Lippenstifte, Antiaknestifte, Sonnenschutzstifte und Lidschattenstifte nach Anspruch 1 oder Verfahren zur Herstellung kosmetischer Stifte nach Anspruch 2, **dadurch gekennzeichnet, dass** der W/O-Emulgator oder die W/O-Emulgatoren gewählt werden aus der Gruppe der Substanzen der allgemeinen Formel 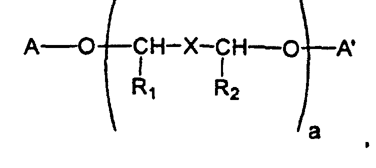 wobei
- A und A' gleiche oder verschiedene hydrophobe organische Reste darstellen,
- a eine Zahl von 1 bis 100, vorzugsweise 2 bis 60, insbesondere 5 bis 40 darstellt,
- X eine Einfachbindung oder die Gruppe 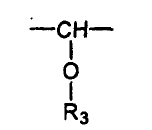
- darstellt,
- R₁ und R₂ unabhängig voneinander so gewählt werden H, Methyl, dass aber nicht beide Reste gleichzeitig Methyl darstellen,
- R₃ gewählt wird aus der Gruppe H, sowie der verzweigten und unverzweigten, gesättigten und ungesättigten Alkyl- und Acylreste mit 1 - 20 Kohlenstoffatomen,
oder dass der oder die W/O-Emulgatoren gewählt werden aus der Gruppe der Fettalkohole mit 8 - 30 Kohlenstoffatomen, Monoglycerinester gesättigter und/oder ungesättigter, verzweigter und/oder unverzweigter Alkancarbonsäuren einer Kettenlänge von 8 - 24, insbesondere 12 - 18 C-Atomen, Diglycerinester gesättigter und/oder ungesättigter, verzweigter und/oder unverzweigter Alkancarbonsäuren einer Kettenlänge von 8 - 24, insbesondere 12 - 18 C-Atomen, Triglycerinester gesättigter und/oder ungesättigter, verzweigter und/oder unverzweigter Alkancarbonsäuren einer Kettenlänge von 8 - 24, insbesondere 12 - 18 C-Atomen, Polyglycerinester gesättigter und/oder ungesättigter, verzweigter und/oder unverzweigter Alkancarbonsäuren einer Kettenlänge von 8 - 24, insbesondere 12 - 18 C-Atomen mit bis zu 10 Glycerineinheiten, Monoglycerinether gesättigter und/oder ungesättigter, verzweigter und/oder unverzweigter Alkohole einer Kettenlänge von 8 - 24, insbesondere 12 - 18 C-Atomen, Diglycerinether gesättigter und/oder ungesättigter, verzweigter und/oder unverzweigter Alkohole einer Kettenlänge von 8 - 24, insbesondere 12 - 18 C-Atomen, Triglycerinether gesättigter und/oder ungesättigter, verzweigter und/oder unverzweigter Alkohole einer Kettenlänge von 8 - 24, insbesondere 12 - 18 C-Atomen, Polyglycerinether gesättigter und/oder ungesättigter, verzweigter und/oder unverzweigter Alkancarbonsäuren einer Kettenlänge von 8 - 24, insbesondere 12 - 18 C-Atomen mit bis zu 10 Glycerineinheiten, Propylenglycolester gesättigter und/oder ungesättigter, verzweigter und/oder unverzweigter Alkancarbonsäuren einer Kettenlänge von 8 - 24, insbesondere 12 - 18 C-Atomen, Sorbitanester gesättigter und/oder ungesättigter, verzweigter und/oder unverzweigter Alkancarbonsäuren einer Kettenlänge von 8 - 24, insbesondere 12 - 18 C-Atomen, Sorbitanester von Polyolen, insbesondere des Glycerins, Pentaerythritylester gesättigter und/oder ungesättigter, verzweigter und/oder unverzweigter Alkancarbonsäuren einer Kettenlänge von 8 - 24, insbesondere 12 - 18 C-Atomen, Methylglucose Ester gesättigter und/oder ungesättigter, verzweigter und/oder unverzweigter Alkancarbonsäuren e iner Kettenlänge von 8 - 24, insbesondere 12 - 18 C-Atomen, Polyglycerin Methylglucose Ester gesättigter und/oder ungesättigter, verzweigter und/oder unverzweigter Alkancarbonsäuren einer Kettenlänge von 8 - 24, insbesondere 12 - 18 C-Atomen
oder dass die vorstehend genannten Typen von W/O-Emulgatoren zusätzlich in der Weise polyethoxyliert und/oder polypropoxyliert sind, dass sie ethoxylierte und/oder propopoxylierte W/O-Emulgatoren darstellen.

4. Lippenstifte, Antiaknestifte, Sonnenschutzstifte und Lidschattenstifte nach Anspruch 3 oder Verfahren zur Herstellung kosmetischer Stifte nach Anspruch 3, **dadurch gekennzeichnet, dass** der W/O-Emulgator oder d ie W/O-Emulgatoren so gewählt werden, dass die Reste A und A' werden vorteilhaft gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema. 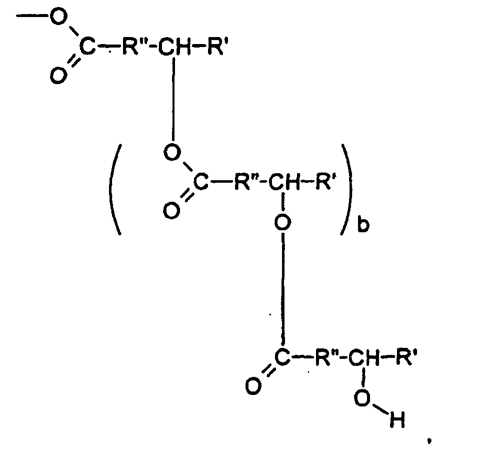 wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann.

5. Lippenstifte, Antiaknestifte, Sonnenschutzstifte und Lidschattenstifte nach Anspruch 1 oder Verfahren zur Herstellung kosmetischer Stifte nach Anspruch 2, **dadurch gekennzeichnet, dass** der oder die W/O-Emulgatoren gewählt werden aus der Gruppe PEG-30 Dipolyhydroxystearat, Decaglycerylheptaoleat, Polyglyceryl-3-Diisostearat, PEG-8 Distearat, Diglycerin Dipolyhydroxystearat.

6. Lippenstifte, Antiaknestifte, Sonnenschutzstifte und Lidschattenstifte nach Anspruch 1 oder Verfahren zur Herstellung kosmetischer Stifte nach Anspruch 2, **dadurch gekennzeichnet, dass** der oder die Stabilisatoren gewählt wird aus der Gruppe der Substanzen der allgemeinen Formel 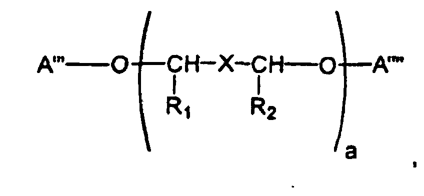 wobei
- A"' und A"" gleiche oder verschiedene hydrophobe organische Reste darstellen,
- a eine Zahl von 1 bis 100, vorzugsweise 2 bis 60, darstellt,
- X eine Einfachbindung oder die Gruppe 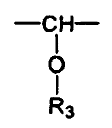
- darstellt,
- R₁ und R₂ unabhängig voneinander aus der Gruppe H, Methyl gewählt werden, dass aber nicht beide Reste gleichzeitig Methyl darstellen,
- R₃ gewählt wird aus der Gruppe H, sowie der verzweigten und unverzweigten, gesättigten und ungesättigten Alkyl- und Acylreste mit 1 - 20 Kohlenstoffatomen,
- wobei die Reste A"' und A"" können gleich oder verschieden sein und gewählt werden aus der Gruppe 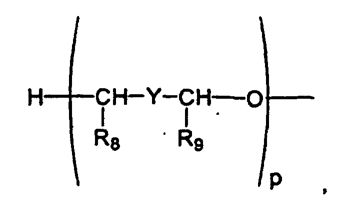
- wobei R₈ und R₉ gleich oder verschieden sein können und gewählt werden aus der Gruppe der gesättigten und ungesättigten Alkyl- und Acylreste mit 1 - 30 Kohlenstoffatomen, p eine Zahl von 1 - 20 darstellt und Y eine Einfachbindung oder die Gruppe 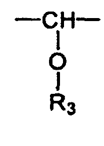 darstellt,
- wobei R₃ gewählt wird aus der Gruppe H, sowie der verzweigten und unverzweigten, gesättigten und ungesättigten Alkyl- und Acylreste mit 1 - 30 Kohlenstoffatomen.
Ferner können die Gruppe A''' und A'''' unabhängig voneinander auch Alkylreste oder Acylreste darstellen.

7. Lippenstifte, Antiaknestifte, Sonnenschutzstifte und Lidschattenstifte nach Anspruch 6 oder Verfahren zur Herstellung kosmetischer Stifte nach Anspruch 6, **dadurch gekennzeichnet, dass** als Stabilisator das PEG-45 /Dodecylglycolcopolymer und/oder das PEG-22 / Dodecylglycolcopolymer und/oder das Methoxy PEG-22/Dodecyl Glycol Copolymer verwendet werden.

8. Lippenstifte, Antiaknestifte, Sonnenschutzstifte und Lidschattenstifte nach Anspruch 1 oder Verfahren zur Herstellung kosmetischer Stifte nach Anspruch 2, **dadurch gekennzeichnet, dass** die Ölkomponente oder die Gesamtheit der Ölkomponenten der erfindungsgemäßen wasserreichen Stifte gewählt wird aus der Gruppe der Ester aus gesättigten und/oder ungesättigten, verzweigten und/oder unverzweigten Alkancarbonsäuren einer Kettenlänge von 14 bis 44 C-Atomen und gesättigten und/oder ungesättigten, verzweigten und/oder unverzweigten Alkoholen einer Kettenlänge von 14 bis 44 C-Atomen, aus der Gruppe der Ester aus aromatischen Carbonsäuren und gesättigten und/oder ungesättigten, verzweigten und/oder u nverzweigten A lkoholen e iner K ettenlänge von 3 b is 3 0 C -Atomen sofern die Ölkomponente oder die Gesamtheit der Ölkomponenten bei Raumtemperatur eine Flüssigkeit darstellen.

9. Lippenstifte, Antiaknestifte, Sonnenschutzstifte und Lidschattenstifte nach Anspruch 1 oder Verfahren zur Herstellung kosmetischer Stifte nach Anspruch 2, **dadurch gekennzeichnet, dass** die Ölkomponente oder die Gesamtheit der Ölkomponenten der erfindungsgemäßen wasserreichen Stifte gewählt wird aus der Gruppe der verzweigten und unverzweigten Kohlenwasserstoffe, der cyclischen oder linearen Silikonöle, der Dialkylether, der Gruppe der gesättigten oder ungesättigten, verzweigten Alkohole, sowie der Fettsäuretriglyceride, namentlich der synthetischen oder natürlichen Triglycerinester gesättigter und/oder ungesättigter, verzweigter und/oder unverzweigter Alkancarbonsäuren einer Kettenlänge von 8 bis 24, insbesondere 12 - 18 C-Atomen,

10. Lippenstifte, Antiaknestifte, Sonnenschutzstifte und Lidschattenstifte nach Anspruch 1 oder Verfahren zur Herstellung kosmetischer Stifte nach Anspruch 2, **dadurch gekennzeichnet, dass** die Wachskomponente oder die Gesamtheit der Wachskomponenten der erfindungsgemäßen W/O-Emulsionsstifte gewählt wird aus der Gruppe der Ester aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, aus der Gruppe der Ester aus aromatischen Carbonsäuren und gesättigten und/oder ungesättigten, verzweigten u nd/oder u nverzweigten A Ikoholen e iner K ettenlänge v on 1 b is 8 0 C -Atomen sofern die Wachskomponente oder die Gesamtheit der Wachskomponenten bei Raumtemperatur einen Festkörper darstellen.

11. Lippenstifte, Antiaknestifte, Sonnenschutzstifte und Lidschattenstifte nach Anspruch 1 oder Verfahren zur Herstellung kosmetischer Stifte nach Anspruch 2, durch einen zusätzlichen Gehalt an einem oder mehreren wasserlöslichen und/oder mit Wasser quellbaren Polymeren gekennzeichnet, insbesondere mit Alkylgruppen veretherte Celluloseund/oder Stärkederivate, bevorzugt β-Glucane, Xanthangummi, Dextrane, Hydroxymethylcellulose, Hydroxyethylcellulose und/oder Hydroxypropylcellulose, Methoxy-PEG-22/ Dodecyl-Glycol-Copolymere, Poloxamere, mit einem oder mehreren n-Octenylsuccinatresten veresterter hydrophiler Stärke.

## Claims

1. Cosmetic sticks, in particular those chosen from the group consisting of lipsticks, antiacne sticks, sunscreen sticks and eyeshadow sticks, **characterized in that** they comprise
(a) a lipid phase, which comprises
(a1) at least one oil component
(a2) at least one wax component
(a3) optionally other substances soluble or dispersible in the lipid phase,
(b) an aqueous phase, which comprises
(b1) from 50 to 85% by weight of water, based on the total weight of the stick composition and
(b2) if desired, substances soluble or dispersible in water;
(c) if desired, one or more active ingredients, chosen from the active ingredients known for lipsticks, antiacne sticks, sunscreen sticks and eyeshadow sticks,
(d) at least one W/O emulsifier or a mixture of two or more W/O emulsifiers,
(e) one or more stabilizers, chosen from the group of substances of the general structure A-B-A', where A and A' are identical or different hydrophobic organic radicals, and B is a hydrophilic group,
(f) if desired, further surface-active substances as coemulsifiers, and also, if desired, further stabilizers and further customary cosmetic and/or pharmaceutical auxiliaries, active ingredients and/or additives.

2. Process for preparing cosmetic sticks, in particular those chosen from the group consisting of lipsticks, antiacne sticks, sunscreen sticks and eyeshadow sticks, which are **characterized in that** they comprise
(a) a lipid phase, which comprises
(a1) at least one oil component
(a2) at least one wax component
(a3) optionally other substances soluble or dispersible in the lipid phase,
(b) an aqueous phase, which comprises
(b1) from 30 to 85% by weight of water, based on the total weight of the stick composition and
(b2) if desired, substances soluble or dispersible in water,
(c) at least one or more active ingredient or several chosen from the active ingredients known for lipsticks, antiacne sticks, sunscreen sticks and eyeshadow sticks,
(d) at least one W/O emulsifier or a mixture of two or more W/O emulsifiers,
(e) one or more stabilizers, chosen from the group of substances of the general structure A-B-A', where A and A' are identical or different hydrophobic organic radicals, and B is a hydrophilic group,
(f) if desired, further surface-active substances as coemulsifiers, and also, if desired, stabilizers and further customary cosmetic and/or pharmaceutical auxiliaries, active ingredients and/or additives,
which is **characterized in that** the water phase and the heated lipid phase are added together in a one-step process and then cooled to room temperature.

3. Lipsticks, antiacne sticks, sunscreen sticks and eyeshadow sticks according to Claim 1 or process for preparing cosmetic sticks according to Claim 2, **characterized in that** the W/O emulsifier or the W/O emulsifiers are chosen from the group of substances of the general formula 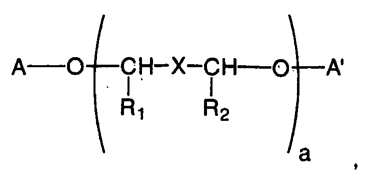 where
- A and A' are identical or different hydrophobic organic radicals,
- a is a number from 1 to 100, preferably from 2 to 60, in particular from 5 to 40,
- X is a singie bond or the group 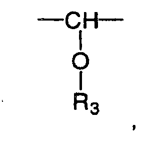
- R₁ and R₂ independently of one another are chosen from the group consisting of H or methyl, but such that the two radicals are not methyl at the same time,
- R₃ is chosen from the group consisting of H and the branched and unbranched, saturated and unsaturated alkyl and acyl radicals having 1 - 20 carbon atoms,
or that the W/O emulsifier(s) is/are chosen from the group of fatty'alcohols having 8 - 30 carbon atoms, monoglycerol esters of saturated and/or unsaturated, branched and/or unbranched alkanecarboxylic acids having a chain length of 8 - 24, in particular 12 - 18, carbon atoms, diglycerol esters of saturated and/or unsaturated, branched and/or unbranched alkanecarboxylic acids having a chain length of 8 - 24, in particular 12 - 18, carbon atoms, triglycerol esters of saturated and/or unsaturated, branched and/or unbranched alkanecarboxylic acids having a chain length of 8 - 24, in particular 12 - 18, carbon atoms, polyglycerol esters of saturated and/or unsaturated, branched and/or unbranched alkanecarboxylic acids having a chain length of 8 - 24, in particular 12 - 18, carbon atoms and up to 10 glycerol units, monoglycerol ethers of saturated and/or unsaturated, branched and/or unbranched alcohols having a chain length of 8 - 24, in particular 12 - 18, carbon atoms, diglycerol ethers of saturated and/or unsaturated, branched and/or unbranched alcohols having a chain length of 8 - 24, in particular 12 - 18, carbon atoms, triglycerol ethers of saturated and/or unsaturated, branched and/or unbranched alcohols having a chain length of 8 - 24, in particular 12 - 18, carbon atoms, polyglycerol ethers of saturated and/or unsaturated, branched and/or unbranched alkanecarboxylic acids having a chain length of 8 - 24, in particular 12 - 18, carbon atoms and up to 10 glycerol units, propylene glycol esters of saturated and/or unsaturated, branched and/or unbranched alkanecarboxylic acids having a chain length of 8 - 24, in particular 12 - 18, carbon atoms, sorbitan esters of saturated and/or unsaturated, branched and/or unbranched alkanecarboxylic acids having a chain length of 8 - 24, in particular 12 - 18, carbon atoms, sorbitan esters of polyols, in particular of glycerol, pentaerythritol esters of saturated and/or unsaturated, branched and/or unbranched alkanecarboxylic acids having a chain length of 8 - 24, in particular 12 - 18, carbon atoms, methylglucose esters of saturated and/or unsaturated, branched and/or unbranched alkanecarboxylic acids having a chain length of 8 - 24, in particular 12 - 18, carbon atoms, polyglycerol methylglucose esters of saturated and/or unsaturated, branched and/or unbranched alkanecarboxylic acids having a chain length of 8 - 24, in particular 12 - 18, carbon atoms
or that the abovementioned types of W/O emulsifiers are additionally polyethoxylated and/or polypropoxylated such that they are ethoxylated and/or propoxylated W/O emulsifers.

4. Lipsticks, antiacne sticks, sunscreen sticks and eyeshadow sticks according to Claim 3 or process for preparing cosmetic sticks according to Claim 3, **characterized in that** the W/O emulsifier or the W/O emulsifiers is/are chosen such that the radicals A and A' are advantageously chosen from the group of branched and unbranched, saturated and unsaturated alkyl and acyl radicals and hydroxyacyl radicals having 10 - 30 carbon atoms and also from the group of hydroxyacyl groups joined together via ester functions, according to the formula 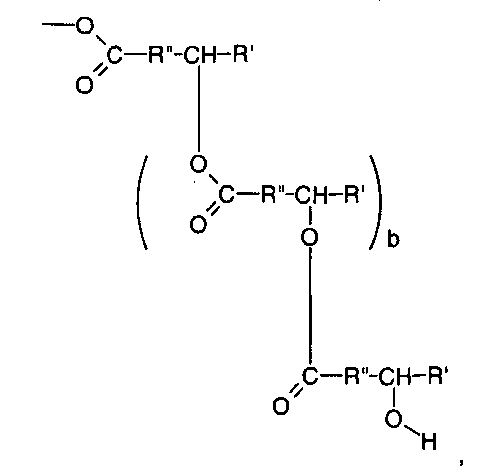 where R' is chosen from the group of branched and unbranched alkyl groups having from 1 to 20 carbon atoms, and R" is chosen from the group of branched and unbranched alkylene groups having from 1 to 20 carbon atoms, and b can assume numbers from 0 to 200.

5. Lipsticks, antiacne sticks, sunscreen sticks and eyeshadow sticks according to Claim 1 or process for preparing cosmetic sticks according to Claim 2, **characterized in that** the W/O emulsifier(s) is/are chosen from the group consisting of PEG-30 dipolyhydroxystearate, decaglyceryl heptaoleate, polyglyceryl-3 diisostearate, PEG-8 distearate, diglycerol dipolyhydroxystearate.

6. Lipsticks, antiacne sticks, sunscreen sticks and eyeshadow sticks according to Claim 1 or process for preparing cosmetic sticks according to Claim 2, **characterized in that** the stabilizer(s) is/are chosen from the group of substances of the general formula 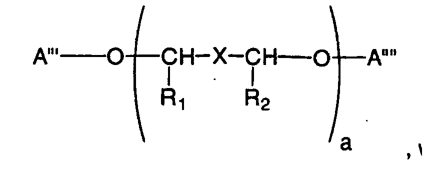 where
- A"' and A"" are identical or different hydrophobic organic radicals,
- a is a number from 1 to 100, preferably from 2 to 60,
- X is a single bond or the group 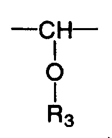
- R₁ and R₂ independently of one another are chosen from the group consisting of H and methyl, but such that the two radicals are not methyl at the same time,
- R₃ is chosen from the group consisting of H and the branched and unbranched, saturated and unsaturated alkyl and acyl radicals having 1 - 20 carbon atoms,
- where the radicals A''' and A'''' can be identical or different and are chosen from the group 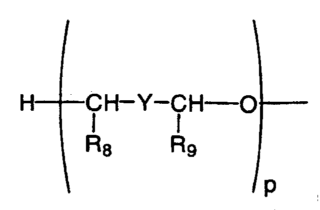
- where R₈ and R₉ can be identical or different and are chosen from the group of saturated and unsaturated alkyl and acyl radicals having 1 - 30 carbon atoms, p is a number from 1 to 20, and Y is a single bond or the group 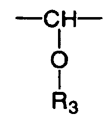
where R₃ is chosen from the group consisting of H and the branched and unbranched, saturated and unsaturated alkyl and acyl radicals having 1 - 30 carbon atoms.
In addition, the group A''' and A'''' can, independently of one another, also be alkyl radicals or acyl radicals.

7. Lipsticks, antiacne sticks, sunscreen sticks and eyeshadow sticks according to Claim 6 or process for preparing cosmetic sticks according to Claim 6, **characterized in that** the stabilizer used is the PEG-45/dodecyl glycol copolymer and/or the PEG-22/dodecyl glycol copolymer and/or the methoxy-PEG-22/dodecyl glycol copolymer.

8. Lipsticks, antiacne sticks, sunscreen sticks and eyeshadow sticks according to Claim 1 or process for preparing cosmetic sticks according to Claim 2, **characterized in that** the oil component or the totality of the oil components of the novel water-rich sticks is chosen from the group of esters of saturated and/or unsaturated, branched and/or unbranched alkanecarboxylic acids having a chain length of from 14 to 44 carbon atoms and saturated and/or unsaturated, branched and/or unbranched alcohols having a chain length of from 14 to 44 carbon atoms, from the group of esters of aromatic carboxylic acids and saturated and/or unsaturated, branched and/or unbranched alcohols having a chain length of from 3 to 30 carbon atoms, provided the oil component or the totality of the oil components is a liquid at room temperature.

9. Lipsticks, antiacne sticks, sunscreen sticks and eyeshadow sticks according to Claim 1 or process for preparing cosmetic sticks according to Claim 2, **characterized in that** the oil component or the totality of the oil components of the novel water-rich sticks is chosen from the group of branched and unbranched hydrocarbons, cyclic or linear silicone oils, dialkyl ethers, the group of saturated or unsaturated, branched alcohols, and the fatty acid triglycerides, namely the synthetic or natural triglycerol esters of saturated and/or unsaturated, branched and/or unbranched alkanecarboxylic acids having a chain length of from 8 to 24, in particular 12 - 18, carbon atoms.

10. Lipsticks, antiacne sticks, sunscreen sticks and eyeshadow sticks according to Claim 1 or process for preparing cosmetic sticks according to Claim 2, **characterized in that** the wax component or the totality of the wax components of the novel W/O emulsion sticks is chosen from the group of esters of saturated and/or unsaturated, branched and/or branched alkanecarboxylic acids having a chain length of from 1 to 80 carbon atoms and saturated and/or unsaturated, branched and/or unbranched alcohols having a chain length of from 1 to 80 carbon atoms, from the group of esters of aromatic carboxylic acids and saturated and/or unsaturated, branched and/or unbranched alcohols having a chain length of from 1 to 80 carbon atoms, provided that the wax component or the totality of the wax components is a solid at room temperature.

11. Lipsticks, antiacne sticks, sunscreen sticks and eyeshadow sticks according to Claim 1 or process for preparing cosmetic sticks according to Claim 2, **characterized by** an additional content of one or more water-soluble and/or water-swellable polymers, in particular alkyl-etherified cellulose and/or starch derivatives, preferably β-glucans, xanthan gum, dextrans, hydroxymethylcellulose, hydroxyethylcellulose and/or hydroxypropylcellulose, methoxy PEG-22/ dodecyl glycol copolymers, poloxamers, hydrophilic starch esterified with one or more n-octenylsuccinate radicals.

## Revendications

1. Bâtons cosmétiques, en particulier ceux choisis dans le groupe des bâtons pour les lèvres, bâtons antiacnéiques, bâtons antisolaires et bâtons d'ombre à paupières, **caractérisés en ce qu'**ils contiennent
(a) une phase lipidique qui comprend
(a1) au moins un composant huileux
(a2) au moins un composant de type cire
(a3) éventuellement d'autres substances solubles ou dispersables dans la phase lipidique,
(b) une phase aqueuse qui comprend
(b1) 50 à 85 % en poids d'eau, par rapport au poids total de la matière de remplissage du bâton, ainsi que
(b2) si on le désire des substances solubles ou dispersables dans l'eau,
(c) si on le désire une ou plusieurs substances actives choisies parmi les substances actives connues pour des bâtons pour les lèvres, des bâtons antiacnéiques, des bâtons photoprotecteurs et des bâtons d'ombre à paupières,
(d) au moins un émulsifiant E/H ou un mélange de plusieurs émulsifiants E/H,
(e) un ou plusieurs stabilisants, choisis dans le groupe des substances de structure générale A-B-A', A et A' représentant des radicaux organiques hydrophobes identiques ou différents et B signifiant un groupe hydrophile,
(f) si on le désire d'autres substances tensioactives en tant que co-émulsifiants, en outre, si on le désire d'autres stabilisants et d'autres actifs, additifs et/ou adjuvants cosmétiques et/ou pharmaceutiques usuels.

2. Procédé pour la fabrication de bâtons cosmétiques, en particulier de ceux choisis dans le groupe des bâtons pour les lèvres, bâtons antiacnéiques, bâtons antisolaires et bâtons d'ombre à paupières, qui sont **caractérisés en ce qu'**ils contiennent.
(a) une phase lipidique qui comprend
(a1) au moins un composant huileux
(a2) au moins un composant de type cire
(a3) éventuellement d'autres substances solubles ou dispersables dans la phase lipidique,
(b) une phase aqueuse qui comprend
(b1) 30 à 85 % en poids d'eau, par rapport au poids total de la matière de remplissage du bâton, ainsi que
(b2) si on le désire des substances solubles ou dispersables dans l'eau,
(c) si on le désire une ou plusieurs substances actives choisies parmi les substances actives connues pour des bâtons pour les lèvres, des bâtons antiacnéiques, des bâtons photoprotecteurs et des bâtons d'ombre à paupières,
(d) au moins un émulsifiant E/H ou un mélange de plusieurs émulsifiants E/H,
(e) un ou plusieurs stabilisants, choisis dans le groupe des substances de structure générale A-B-A', A et A' représentant des radicaux organiques hydrophobes identiques ou différents et B signifiant un groupe hydrophile,
(f) si on le désire d'autres substances tensioactives en tant que co-émulsifiants, en outre, si on le désire d'autres stabilisants et d'autres actifs, additifs et/ou adjuvants cosmétiques et/ou pharmaceutiques usuels,
qui est **caractérisé en ce que** la phase aqueuse et la phase lipidique chauffée sont réunies dans un procédé en une seule étape et ensuite refroidies jusqu'à la température ambiante.

3. Bâtons pour les lèvres, bâtons antiacnéiques, bâtons photoprotecteurs et bâtons d'ombre à paupières selon la revendication 1, ou procédé pour la fabrication de bâtons cosmétiques selon la revendication 2,
**caractérisés en ce que** l'émulsifiant E/H ou les émulsifiants E/H sont choisis dans le groupe des substances de formule générale 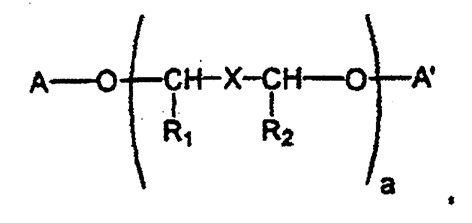 dans laquelle
- A et A' représentent des radicaux organiques hydrophobes identiques ou différents,
- a représente un nombre allant de 1 à 100, dé préférence de 2 à 60, en particulier de 5 à 40,
- X représente une liaison simple ou le groupe 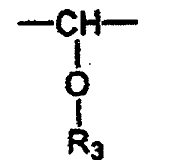
- R₁ et R₂ sont choisis, indépendamment l'un de l'autre, parmi H et le groupe méthyle, mais les deux radicaux ne peuvent pas représenter simultanément le groupe méthyle,
- R₃ est choisi parmi H et des radicaux alkyle et acyle ramifiés ou non ramifiés, saturés ou non saturés, ayant de 1 à 20 atomes de carbone,
ou **en ce que** l'émulsifiant ou les émulsifiants E/H sont choisis dans le groupe des alcools gras ayant de 8 à 30 atomes de carbone, des esters de monoglycérol avec des acides alcanecarboxyliques saturés et/ou insaturés, ramifiés et/ou non ramifiés, ayant une longueur de chaîne de 8-24, en particulier 12-18 atomes de carbone, des esters de diglycérol avec des acides alcanecarboxyliques saturés et/ou insaturés, ramifiés et/ou non ramifiés, ayant une longueur de chaîne de 8-24, en particulier 12-18 atomes de carbone, des esters de triglycérol avec des acides alcanecarboxyliques saturés et/ou insaturés, ramifiés et/ou non ramifiés, ayant une longueur de chaîne de 8-24, en particulier 12-18 atomes de carbone, des esters de polyglycérol avec des acides alcanecarboxyliques saturés' et/ou insaturés, ramifiés et/ou non ramifiés, ayant une longueur de chaîne de 8-24, en particulier 12-18 atomes de carbone et comportant jusqu'à 10 groupes glycérol, des éthers de monoglycérol avec des alcools saturés et/ou insaturés, ramifiés et/ou non ramifiés, ayant une longueur de chaîne de 8-24, en particulier 12-18 atomes de carbone, des éthers de diglycérol avec des alcools saturés et/ou insaturés, ramifiés et/ou non ramifiés, ayant une longueur de chaîne de 8-24, en particulier 12-18 atomes de carbone, des éthers de triglycérol avec des alcools saturés et/ou insaturés, ramifiés et/ou non ramifiés, ayant une longueur de chaîne de 8-24, en particulier 12-18 atomes de carbone, des éthers de polyglycérol avec des acides alcanecarboxyliques saturés et/ou insaturés, ramifiés et/ou non ramifiés, ayant une longueur de chaîne de 8-24, en particulier 12-18 atomes de carbone et comportant jusqu'à 10 groupes glycérol, des esters de propylèneglycol avec des acides alcanecarboxyliques saturés et/ou insaturés, ramifiés et/ou non ramifiés, ayant une longueur dé chaîne de 8-24, en particulier 12-18 atomes de carbone, des esters de sorbitanne avec des acides alcanecarboxyliques saturés et/ou insaturés, ramifiés et/ou non ramifiés, ayant une longueur de chaîne de 8-24, en particulier 12-18 atomes de carbone, des esters de sorbitanne avec des polyols, en particulier avec le glycérol, des esters de pentaérythritol avec des acides alcanecarboxyliques saturés et/ou insaturés, ramifiés et/ou non ramifiés, ayant une longueur de chaîne de 8-24, en particulier 12-18 atomes de carbone, des esters de méthylglucose avec des acides alcanecarboxyliques saturés et/ou insaturés, ramifiés et/ou non ramifiés, ayant une longueur de chaîne de 8-24, en particulier 12-18 atomes de carbone, des esters de polyglycérol-méthylglucose avec des acides alcanecarboxyliques saturés et/ou insaturés, ramifiés et/ou non ramifiés, ayant une longueur de chaîne de 8-24, en particulier 12-18 atomes de carbone, ou **en ce que** les types précités d'émulsifiants E/H sont en outre polyéthoxylés et/ou polypropoxylés, de sorte qu'ils représentent des émulsifiants E/H éthoxylés et/ou propoxylés.

4. Bâtons pour les lèvres, bâtons antiacnéiques, bâtons antisolaires et bâtons d'ombre à paupières selon la revendication 3 ou procédé pour la fabrication de bâtons cosmétiques selon la revendication 3, **caractérisés en ce que** l'émulsifiant E/H ou les émulsifiants E/H sont choisis de sorte que les radicaux A et A' sont choisis avantageusement dans le groupe des radicaux alkyle et acyle ramifiés ou non ramifiés, saturés ou insaturés, ayant de 10 à 30 atomes de carbone, ainsi qu'en outre dans l'ensemble des groupes hydrbxyacyle reliés entre eux par des fonctions ester, selon le schéma 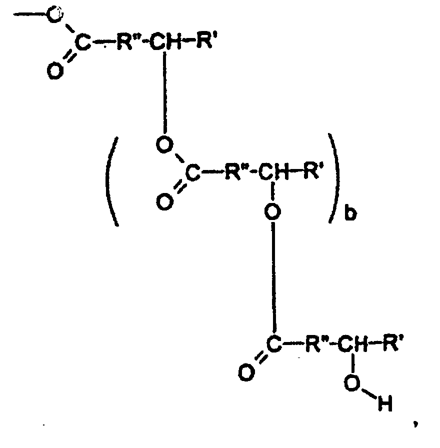 R' étant choisi dans l'ensemble constitué par des groupes alkyle ramifiés ou non ramifiés ayant de 1 à 20 atomes de carbone, et R" étant choisi dans l'ensemble constitué par les groupes alkylène ramifiés ou non ramifiés ayant de 1 à 20 atomes de carbone, et b pouvant représenter des nombres allant de 0 à 200.

5. Bâtons pour les lèvres, bâtons antiacnéiques, bâtons antisolaires et bâtons d'ombre à paupières selon la revendication 1 ou procédé pour la fabrication de bâtons cosmétiques selon la revendication 2, **caractérisés en ce que** l'émulsifiant ou l'es émulsifiants E/H sont choisis dans le groupe constitué par le dipolyhydroxystéarate de PEG-30, l'héptaoléate de décaglycéryle, le 3-diisostéarate de polyglycéryle, le distéarate de PEG-8, le dipolyhydroxystéarate de diglycérol.

6. Bâtons pour les lèvres, bâtons antiacnéiques, bâtons antisolaires et bâtons d'ombre à paupières selon la revendication 1 ou procédé pour la fabrication de bâtons cosmétiques selon la revendication 2, **caractérisés en ce que** le ou les stabilisants sont choisis dans le groupe des substances de formule générale 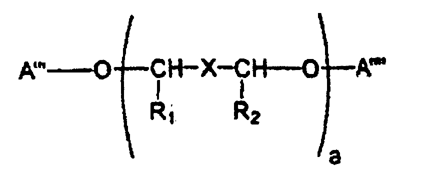 dans laquelle
- A"' et A"" représentent des radicaux organiques hydrophobes identiques ou différents,
- a représente un nombre allant de 1 à 100, de préférence de 2 à 60,
- X représente une liaison simple ou le groupe 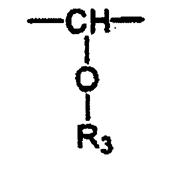
- R₁ et R₂ sont choisis, indépendamment l'un de l'autre, parmi H et le groupe méthyle, mais les deux radicaux ne représentent pas simultanément le groupe méthyle,
- R₃ est choisi dans le groupe constitué par H et des radicaux alkyle et acyle ramifiés ou non ramifiés, saturés ou non saturés, ayant de 1 à 20 atomes de carbone,
- les restes A"' et A"" pouvant être identiques ou différents et sont choisis de préférence dans le groupe 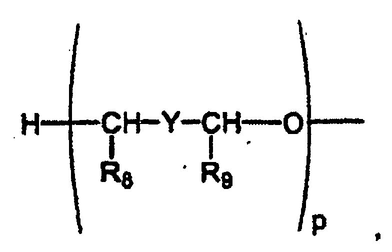
- dans lequel R₈ et R₉ peuvent être identiques ou différents et sont choisis dans l'ensemble des radicaux alkyle et acyle saturés ou insaturés ayant de 1 à 30 atomes de carbone, p représente un nombre allant de 1 à 20 et Y représente une liaison simple ou le groupe 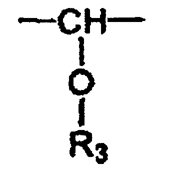
- R₃ étant choisi dans le groupe constitué par H et des radicaux alkyle et acyle ramifiés ou non ramifiés, saturés ou non saturés, ayant de 1 à 30 atomes de carbone,
- les groupes A"' et A"" pouvant en outre représenter également, indépendamment l'un de l'autre, des radicaux alkyle ou des radicaux acyle.

7. Bâtons pour les lèvres, bâtons antiacnéiques, bâtons antisolaires et bâtons d'ombre à paupières selon la revendication 6 ou procédé pour la fabrication de bâtons cosmétiques selon la revendication 6, **caractérisés en ce qu'**on utilise en tant que stabilisant le copolymère PEG-45/dodécylglycol et/ou le copolymère PEG-22/dodécylglycol et/ou le copolymère méthoxy-PEG-22/dodécylglycol.

8. Bâtons pour les lèvres, bâtons antiacnéiques, bâtons antisolaires et bâtons d'ombre à paupières selon la revendication 1 ou procédé pour la fabrication de bâtons cosmétiques selon là revendication 2, **caractérisés en ce que** le composant huileux ou la totalité des composants huileux des bâtons à forte teneur en eau selon l'invention sont choisis dans le groupe des esters d'acides alcanecarboxyliques saturés et/ou insaturés, ramifiés et/ou non ramifiés, ayant une longueur de chaîne de 14 à 44 atomes de carbone, et d'alcools saturés et/ou insaturés, ramifiés et/ou non ramifiés, ayant une longueur de chaîne de 14 à 44 atomes de carbone, dans le groupe des esters d'acides carboxyliques aromatiques et d'alcools saturés et/ou insaturés, ramifiés et/ou non ramifiés, ayant une longueur de 3 à 30 atomes de carbone, lorsque le composant huileux ou la totalité des composants huileux représentent un liquide à la température ambiante.

9. Bâtons pour les lèvres, bâtons antiacnéiques, bâtons antisolaires et bâtons d'ombre à paupières selon la revendication 1 ou procédé pour la fabrication de bâtons cosmétiques selon la revendication 2, **caractérisés en ce que** le composant huileux ou la totalité des composants huileux des bâtons à forte teneur en eau selon l'invention sont choisis dans le groupe des hydrocarbures ramifiés et/ou non ramifiés, des huiles de silicone linéaires ou cycliques, des éthers dialkyliques, dans le groupe des alcools ramifiés saturés et/ou insaturés, ainsi que des triglycérides d'acides gras, à savoir des esters de triglycérol naturels ou synthétiques avec des acides alcanecarboxyliques saturés et/ou insaturés, ramifiés et/ou non ramifiés, ayant une longueur de chaîne de 8 à 24, en particulier de 12 à 18 atomes de carbone.

10. Bâtons pour les lèvres, bâtons antiacnéiques, bâtons antisolaires et bâtons d'ombre à paupières selon la revendication 1 ou procédé pour la fabrication de bâtons cosmétiques selon la revendication 2, **caractérisés en ce que** le composant de type cire ou la totalité des composants de type cire des bâtons d'émulsion E/H selon l'invention sont choisis dans le groupe des esters d'acides alcanecarboxyliques saturés et/ou insaturés, ramifiés et/ou non ramifiés, ayant une longueur de chaîne de 1 à 80 atomes de carbone, et d'alcools saturés et/ou insaturés, ramifiés et/ou non ramifiés, ayant une longueur de chaîne de 1 à 80 atomes de carbone, dans le groupe des esters d'acides carboxyliques aromatiques et d'alcools saturés et/ou insaturés, ramifiés et/ou ou non ramifiés, ayant une longueur de chaîne de 1 à 80 atomes de carbone, lorsque le composant de type cire ou la totalité des composants de type cire représentent un solide à la température ambiante.

11. Bâtons pour les lèvres, bâtons antiacnéiques, bâtons antisolaires et bâtons d'ombre à paupières selon la revendication 1 ou procédé pour la fabrication de bâtons cosmétiques selon la revendication 2, **caractérisés par** une teneur supplémentaire en un ou plusieurs, polymères hydrosolubles et/ou susceptibles de gonfler avec l'eau, en particulier en dérivés d'amidon et/ou de cellulose éthérifiés par des groupes alkyle, de préférence les β-glucanes, la gomme xanthane, les dextranes, l'hydroxyméthylcellulose, l'hydroxyéthylcellulose et/ou l'hydroxypropylcellulose, les copolymères méthoxy-PEG-22/ dodécylglycol, les poloxamères, des amidons hydrophiles estérifiés par un ou plusieurs radicaux n-octénylsuccinate.
